# EUROPEAN PATENT APPLICATION

(11) **EP 4 059 437 A1**
(43) Date of publication of application: **21.09.2022**
(21) Application number: 21162706.2
(22) Date of filing: 15.03.2021
(51) Int. Cl.: A61B 8/00, A61B 8/08, A61B 8/06

(54) **ULTRASOUND SYSTEM**

(71) Applicant: Pulsify Medical, 3001 Leuven (BE)
(72) Inventor: BARBOSA, Daniel, 4710-048 Braga (PT); D'HOOGE, Jan, 2800 Mechelen (BE)
(74) Representative: DTS Patent- und Rechtsanwälte Schnekenbühl und Partner mbB

(57) **Abstract**

The invention relates to an ultrasound system (1), in particular for cardiac monitoring, comprising a transducer array (3) with a plurality of transducer array elements (3a) for providing ultrasound waves; a control unit (40), which is coupled to the transducer array (3), for controlling the transducer array (3), a data processing unit (41) for receiving data from the transducer array (3) and processing the received data, wherein the system further comprises at least one reference module (45) configured for obtaining reference ultrasound data, at least one volume reconstruction module (46) configured for reconstructing the volume of a target organ or part of a target organ, at least one selected monitoring unit (47) configured for monitoring the volume of the target organ and/or part of the target organ. The invention further relates to a method for ultrasound imaging of a body part of a subject.

## Description

The present invention relates to an ultrasound system, in particular a wearable ultrasound monitor system, in particular for cardiac monitoring.

Wearable ultrasound monitor systems monitor cardiac function over time. The ultrasound monitor can be body worn in a patch type format.

US2016/0136462 A1 discloses a wearable ultrasound device and method of using the device including a power controller with a power source and at least one integrated circuit that delivers electrical power to an applicator. The applicator is electrically coupled to the power controller and a surface of the applicator transmits ultrasound to a wearer for a given duration. The applicator includes radio frequency (RF) drive electronics, an ultrasound transducer coupled to the drive electronics, a monitoring apparatus that includes a thermal cutoff coupled to the drive electronics.

WO2018/102828 A1 discloses an ultrasound coupling patch for use with ultrasound transducers, and more particularly to ultrasound coupling patches having a gel capture feature.

WO 2019/162485 A1 discloses a method and an apparatus for performing ultrasound measurements of a propagation medium containing non uniformities, with a transmitter/transducer with a plurality of transmitting elements for emitting ultrasound excitation pressure waves.

In ultrasound systems with a large number of ultrasound transducer elements, e.g., when a transducer array with 10.000 or 100.000 elements is used, individually sampling every single transducer element leads to massive amounts of data. The system needs accordingly high capabilities for the throughput and the processing of this data. A high amount of power is required to drive and read such an array and all its individual transducer elements.

Power consumption is a crucial factor for operating the ultrasound system, especially when the system is configured for long-term monitoring. It is even more important for a portable ultrasound device, which may comprise or be connected to a portable power source with a limited capacity.

It is an object of the present invention to improve an ultrasound system, in particular an ultrasound system for cardiac monitoring, especially in that the ultrasound system is able to function several days on end without needing to recharge or change the battery of the ultrasound system.

This requirement puts severe challenges on the available power and the allowable computing requirements.

This object is solved according to the present invention with an ultrasound system with the features of claim 1. Accordingly, an ultrasound system, in particular for cardiac monitoring, comprising:
- a transducer array with a plurality of transducer array elements for providing ultrasound waves;
- a control unit, which is coupled to the transducer array, for controlling the transducer array,
- a data processing unit for receiving data from the transducer array and processing the received data,
wherein the system further comprises
- at least one reference module configured for obtaining reference ultrasound data,
- at least one volume reconstruction module configured for reconstructing the volume of a target organ or part of a target organ,
- at least one selected monitoring unit configured for monitoring the volume of the target organ and/or part of the target organ.

A wave in sense of this application is to be understood as a transport of energy through a medium, i.e. it can be understood as any kind of ultrasound signal.

Further advantageous embodiments are given in the dependent claims.

In particular, the ultrasound system can be in general an ultrasound system as disclosed by WO 2019/162485 A1. The further disclosed features can be added for example to the system as disclosed in WO 2019/162485 A1.

The invention is based on the basic idea that with focusing monitoring on a feature of interest and/or selected time points instead of constant monitoring of an unrestricted area by the whole transducer array power saving is enabled. The use of a hardware system including a transducer array with a plurality of transducer array elements, a control unit, a data processing unit, a reference module, a volume reconstruction module and a selected monitoring unit may enable to restrict monitoring on the volume of a feature of interest such as an organ, fluid carrying structure or other volumetric feature in the body, e.g. the heart, or a part of a target organ, in particular a ventricle or atrium, which during a cardiac cycle or selected phases of a cardiac cycle provides the required physiological and/or pathological information. For instance, the volume of the left ventricle instead of the volume of the total heart may be monitored to determine how much volume of blood the heart is pumping, in particular per cardiac cycle or a selected phase of a cardiac cycle Overall, through this restriction of monitoring volume, a less power consuming monitoring and a longer lifetime of the battery of the system and/or less frequent charging is enabled. In other words, the system advantageously requires less computational capacities and the power consumption of the ultrasound system is reduced.

The system may be configured for cardiac monitoring, which may for example comprise monitoring of cardiac activity and/or cardiac properties of a patient. Monitoring cardiac activity may comprise monitoring of parameters such as cardiac rhythm and/or cardiac output.

The system has at least one transducer array, i.e. a device or unit comprising a plurality of transducer array elements. In particular, the transducer array may have a chip design. The transducer array elements are units, which may be used as an actuator for generating an ultrasound pulse or signal. Alternatively or additionally, the transducer array elements may also be used for receiving or detecting an ultrasound pulse or signal. Generally speaking, one transducer array element may consist of one or more transducer sub-elements. Such a sub-element can be a single transducer only, which can be a piezoelectric micromachined ultrasonic transducer (pMUT) component or a capacitive micromachined ultrasonic transducer (cMUT) component.

In an embodiment, a transducer array element may for example consist of four, nine, sixteen transducer sub-elements, which may be arranged in either a 2x2, 3x3, 4x4 grid or in another suitable fashion. Also non square configurations can be used e.g. for 6 sub-elements in a 2x3 configuration. In further embodiments, other numbers of transducer sub-elements in one transducer array element can be provided, and the transducer sub-elements of one transducer array element may be arranged in a grid or in another arrangement, for example similar to a crystal lattice arrangement. The sub-elements may be connected either in a series and/or parallel connection and provide a common input and output such that they can be operated as a single functional unit.

Different techniques may be used to manufacture transducer elements and/or sub-elements. For example, cMUT units may be fabricated using a polymer-based technique, such as described by Gerardo et al. in "Fabrication and testing of polymer-based capacitive micromachined ultrasound transducers for medical imaging", (Microsystems & Nanoengineering (2018) 4:19).

In order to reduce the complexity of the system, several transducer elements may be combined into a functional unit and treated as a single "element", i.e., the combined transducer array elements are driven at that same time as if they are a single unit.

The transducer array elements in the transducer array can be connected in series or parallel, such that they can be driven optimally.

In particular, the transducer array elements may be configured such that they are individually controllable. For example, in a 2D array configuration the transducer elements could be connected in parallel in the direction of the rows or columns of the array to create a row/column based architecture.

Also, the system comprises a control unit. The control unit may comprise several components, which may be structurally integrated with each other, for example in a common casing or envelope structure, or which may be implemented as structurally separated components, which may be couple to each other.

The transducer array elements are configured to generate and/or receive ultrasound, in particular ultrasound pulses or signals, based on a control signal. To provide the respective control signals for the transducer array, the control unit may comprise a send module and a receive module, which may be implemented in a driver module for the transducer array and/or for transistors, which may control the transducer array elements.

The control unit may be configured to perform different tasks such as, but not limited to, the following examples: The control unit may be configured to perform digital signal processing and/or filtering. Also, the control unit may be configured to perform operations related to storing and/or retrieving information, in particular in a memory module, such as a random access memory (RAM) module. Also, the control unit may be configured to perform operations for driving an external interface, such as an interface to a memory module, a transceiver unit, e.g., via a USB interface or a wireless interface, and/or receiving control signals from other units. The control unit may also perform operations for controlling the transducer array, e.g., through a switch linked to a transducer array element, such a switch can be a transistor or more specifically a thin-film transistor (TFT) element, or a matrix of such switches/transistors, e.g., in a TFT matrix. The TFT matrix can be a(n) (integral) part in terms of function and/or structure of the control unit. Also, the control unit may be configured to control a transistor switch/transistor/ TFT element connected to a transducer element, to generate delays for generating and/or sensing ultrasound pulses and signals, to define generated ultrasound pulses, beamforming operations, and/or to control power management. Each of said functions may be carried out and/or implemented in an ASIC design individually or in combination. Also, these functions may be implemented as modules of the control unit.

The control unit may comprise a switch, e.g. a transistor or thin-film transistor (TFT), which can be configured to control a transducer array element. In particular, an array of switches/transistors/thin-film transistors may be provided, which is coupled to the transducer array.

A TFT array (or more generally speaking the switching module) of the system is used to control the transducer array. The TFT array or the switching module can be coupled to the transducer array and especially it may be coupled to the input ports of the transducer elements. In particular, the switch array (e.g. TFT array) may be structurally integrated with the transducer array in one component, e.g., in a common casing or envelope structure.

The control unit may consist of or comprise one or several microprocessors. Also, the control unit may comprise one or several application-specific integrated circuit (ASIC). The control unit, in particular a microprocessor of the control unit, may be fully or partially integrated in a printed circuit board (PCB). The control unit may also comprise of control circuitry integrated on the panel for the TFT array, this control circuitry will work in conjunction with but as a separate functional unit compared to the TFT switch elements, which enable the selection of the transducer elements. The PCB might be rigid, flexible, stretchable or a hybrid combination thereof.

The system may comprise a field programmable gate array (FPGA) and/or a digital application-specific integrated circuit (ASIC), configured such that one or more control algorithms for the transducer array are carried out. The FPGA and/or ASIC may be comprised by the control unit.

In an embodiment, the system further comprises a beam former module. The beam former module may be configured to apply a time delay to at least one transducer array element or a plurality of transducer array elements. Different time delays may be applied to different sets of transducer array elements. This allows advantageously easy control for high-resolution measurements, beam focusing and/or beam steering.

In particular, the time delays may be applied, when the transducer array element or the plurality of transducer array elements is used for sending ultrasound pulses and/or receiving ultrasound signals.

The time delay for a transducer array element or a plurality of transducer array elements may be defined relative to controlling other transducer array elements.

Also, the beam former module and/or another unit of the system may be utilized to determine the delay. In particular, the delay is determined for each transducer array element or for a plurality of transducer array elements, to which the determined delay is to be applied.

In particular, the control unit may comprise an ASIC for implementing a beam former control, and/or a design for a signal processing algorithm, e.g., for applying spatial and/or temporal sparsity conditions for sending ultrasound pulses and/or for collecting or providing data (related to the measurements performed by the ultrasound device and the ultrasound pulses and signals). In particular, the beam former control can for example comprise an electronic delay and sum circuit that can apply different delays to a certain number of transducer array elements, for example 8 to 32, e.g. 14, 15, 16, 17 or 18 transducer array elements or functional units comprising several connected transducer array elements (e.g. 14, 15, 16, 17 or 18). It may sum the output of these channels into one single output value. In particular, by applying specific delays to each functional unit of transducer array elements, an ultrasound pulse may be generated in a coordinated fashion such that a specific profile for the ultrasound beam is obtained. Also, elements receiving ultrasound signals may be controlled by a beam former in a coordinated way to provide targeted measurements inside a sample volume. The data processing unit may be implemented in different ways. It may for example comprise a microprocessor. In particular, it may comprise an ASIC design. Also, the data processing unit and the control unit may be implemented as software modules that are run, at least partially, on a common computational unit. For example, a processing unit may read from a memory and execute instructions for performing operations to implement the data processing unit and/or control unit.

Also, the data processing unit may comprise one or several analog-digital converters (ADC) and/or digital-analog converters (DAC). These converters are configured to link analog and digital channels to each other and allow both digital data collection and treatment, and treating analog control and detection signals. A time gain controller may be implemented using a DAC element and/or a different architecture.

In one embodiment, the system further comprises a complexity reduction module, which may be configured to define at least one or a plurality of sub-arrays of transducer array elements and to control the at least one or the plurality sub-arrays as functional units for sending at least one (or more) ultrasound pulse(s) and/or receiving at least one or more ultrasound signal(s).

In one embodiment, the complexity reduction module may be implemented as software module, comprising instructions for computational operations that implement the function of complexity reduction. This software module may in particular be implemented as a module of the control unit. Specifically, the complexity reduction module may be comprised, at least partially, by the control unit and/or by the data processing unit.

The reference module implements functions to obtaining reference ultrasound data. Such functions may impact the generation and/or the detection of ultrasound pulses and signals by the transducer array, or they may influence the data acquisition and/or treatment.

When the transducer array is placed on the body of a subject, it does not have any prior information on the features of the body on which it is placed. It may be possible, that in a first step the transducer array/patch will perform a detailed scanning of the region in its field (total or sparse stepping of the array/ matrix of transducers).

This scanning will allow to detect features of interest, including the location of the organ of interest for monitoring (e.g. heart, ventricles, atria, blood vessels, aorta, lungs, etc.), artifacts etc. Any method known in the art can be used for feature detection (e.g. correlation with known feature, neural networks etc.).

The volume reconstruction module reconstructs the volume of the feature of interest of e.g. a target organ or part of a target organ, such as the heart, left/right ventricles, left/right atria, blood vessels, aorta, lungs, etc.

This software module may in particular be implemented together with the control unit. Specifically, the volume reconstruction module may be comprised, at least partially, by the control unit and/or by the data processing unit. The volume reconstruction module may inter alia implement functions to reduce data and sensor complexity and/or quantity. Such functions may impact the generation and/or the detection of ultrasound pulses and signals by the transducer array, or they may influence the data acquisition and/or treatment.

For reconstructing the volume of the feature of interest e.g. a target organ and/or part of a target organ, a collection of collimated and/or focused and/or steered ultrasound beams is provided by a sub-array of the transducer array. Thus, rather than operating the transducer array as a single whole and using each element as an individual unit, a sub-array scanning architecture may be used, i.e., groups of transducer array elements may be combined to functional units. In particular, neighboring elements may be combined. In particular, ultrasound beams can be collimated and/or focused and/or steered by selecting a subset of transducers of the transducer array, a sub-array. The larger the sub-array the more collimated the ultrasound beam will be and the better the focusing capabilities of the sub-array. The ultrasound beam will "shoot" in the direction of the feature of the volume of interest. The reflected ultrasound signals, coming back from a single collimated beam, can be converted in the control unit into imaging data that will give as information the depth information along a single location (determined by positioning of the sub-array and the angle at which the ultrasound beam is send) of the array. This collimated and/or focused and/or steered beam may preferentially intersect with the volume of interest (e.g. for the ventricle this could be the intersection with the myocardium) or with the boundaries (e.g. the myocardium). By shooting multiple of these beams over random or carefully selected locations over the volume and/or the boundaries of the feature of interest, the volume can be sparsely sampled, and this sparse data can be used to reconstruct the volume.

Also, the sparse data may be used to determine the deformation against a more detailed reference volume which would have been collected in an earlier measurement, i.e. the deformation of the reference volume over time with sparse measurements could be tracked. Also, the imaging data may be used directly to track the deformation directly against a reference image.

In particular, the volume reconstruction module may control, for each sub-array, a location of the sub-array within the plurality of transducer array elements. Also, the volume reconstruction module may control, for each sub-array, a focus depth and/or a steering angle for an ultrasound sending beam profile and/or an ultrasound receiving beam profile implemented by the sub-array of transducer array elements; such a beamforming algorithm may be implemented as a software module for a programmable unit and/or by chip design, e.g., in an ASIC.

The transducer array elements of a sub-array may work as a single functional unit, collecting 1D depth data sets by controlling the sending of an ultrasound pulse and collecting reflected ultrasound signals from a sample, e.g., from inside a patient's body.

Also, the system may be configured to generate 3D spatial information from 1D data sets collected by the transducer array, or one or more sub-arrays of the transducer array.

For controlling a sub-array of transducer array elements in order to perform imaging, and, e.g., for generating 3D spatial information, a positioning of the sub-array position may be determined and controlled within the plurality of transducer array elements. This may be carried out by controlling a signal path between a switching array (e.g. a thin-film transistor (TFT) array), which is arranged to transmit excitation signals to the transducer array elements and/or to transmit reading signals from the transducer array elements, and the control unit of the system, in particular a mixed signal ASIC, which may be arranged on the periphery of the sensor. The control unit, especially in combination with other elements or parts of the overall system such as (but not limited to) the switching (TFT) array, and a respective driver module for the switches (e.g. TFT's), may be configured to selectively switch rows and/or columns of transducer array elements to define an active sub-array and a sub-array location in a larger matrix of the system's transducer array.

In order to control direction and depth of a sub-array's ultrasound wave beam and controlling the ultrasound beam's profile, focusing and/or beam-steering may be used. The beam profile is controlled by applying delays on the individual transducer array elements, or by applying delays on a set of combined transducer array elements which provide a common input and output, i.e. a functional unit.. This may be performed both for sending ultrasound pulses, thereby focusing ultrasound energy at a depth defined by a predetermined focal point and sending the ultrasound energy under a predetermined steering angle. Analog to the sending, the beam profile may be controlled for receiving ultrasound signals, e.g., in order to define a direction and a focal depth for receiving ultrasound signals, e.g., from a predetermined location within a sample.

To collect data in order to generate, e.g., 3D spatial information, the control unit may be configured to control, for each sub-array, the location of the sub-array, as well as the focus depth and/or a steering angle for the sub-array depending on a chosen region of interest, e.g., within a patient's body.

In an embodiment, the system further comprises an artifact determination module configured for identifying and avoiding obstructions to ultrasound signals caused by artifacts, such as air bubbles between the transducer array and the skin of the patient equipped with the system or bones, e.g. ribs. In particular, the artifact determination module may be (also) configured to determine a position for a sub-array within the transducer array such that it is not hindered by impediments for ultrasound beams, e.g., due to ribs of a patient close to air bubbles. In other words, this enables limitation of disturbing signals during ultrasound monitoring.

The selected monitoring unit monitors the volume of the target organ and/or part of the target organ. In particular, the computational load may be further reduced by only performing a detailed scanning of the volume at discrete time instances of the cardiac cycle.

Central to the selected monitoring unit is the fact that not the entire volume of the feature (i.e. target organ or part of a target organ) is traced, and not the entire transducer array is used (thus not all transducer elements are used for tracing).

In particular, the system may further comprise at least one phase determination module configured for determining cardiac cycle phases including at least one of start systolic phase, end systolic phase, start diastolic phase and end diastolic phase. Thus, this may enable that the system can advantageously limit the scanning and/or monitoring on specific phases of the cardiac cycle. In particular for evaluating the stroke volume only the end-diastolic and end-systolic phases are of importance. Therefore, a volume scanning only at those time instances may be desired and enabled by means of the selected monitoring unit.

In particular, to enable this detection the phase determination module may need to detect the end-diastolic and end-systolic phases. Therefore, a single (or a few) collimated and/or focused and/or steered ultrasound beams are send through a carefully chosen position of the volume of interest. This will be done several times through the cardiac cycle so that a good graph can be obtained of the contraction over time (e.g. 40 to 200 "samples" could be taken over the full cardiac cycle). From this samples curve the end-diastolic and end-systolic phases can be detected e.g. by looking at the derivative to determine min/max in the curve, alternatively several cycles could be measured, which would allow for accurate estimation of the end-systolic/diastolic phases. The regularity of the heart rhythm can then allow to determine the next instance of the end-diastolic/end-systolic phases. Another option would be to train a neural network, which takes as input the time series and gives as an output the end-diastolic and systolic phases. Also, a gating may be used on another part of the organs. In addition, an ECG signal may also be combined with the ultrasound measurement to determine the heart cycle.

In particular, this scanning of the volume may be done in a time interval which avoids motion artifacts during the measurement. In case the time interval is too short within a single cardiac cycle there is a triggered measurement is possible in which we collect all necessary data over different cardiac cycles (however taking care that each time we collect points at the same phase of the cardiac cycle and corrected for breathing artifacts).

The reference module, the volume restriction module, the selected monitoring unit, the artifact determination module and/or the phase determination module may be implemented as one or more software modules that run, at least in principle, on a common computational unit. In particular, this one or more software module may inter alia comprise instructions for computational operations that implement the function of obtaining reference ultrasound data, reconstructing the volume of a target organ or part of a target organ or monitoring the volume of the target organ and/or part of a target organ, in particular at defined time pints of a cardiac cycle. This software modules may in particular be implemented together with the control unit. Specifically, the reference module, the volume restriction module, the selected monitoring unit, the artifact determination module and/or the phase determination module may be comprised, at least partially, by the control unit and/or by the data processing unit. In an embodiment of the system, the transducer array is flexible and/or stretchable. Thus, the system can advantageously adjust its geometry to a probe or sample surface, e.g., a patient's body. In particular, the transducer array elements may be flexibly oriented towards each other.

For example, the transducer array elements may be supported by a flexible and/or stretchable substrate such that they are arranged on a flat or curved plane. The substrate may, e.g., comprise a polymer material.

In an embodiment of the system, the transducer array is integrated into an adhesive patch for fixing the transducer array to a patient's body. The system can then advantageously be easily used, e.g., for monitoring a patient's body functions, such as cardiac functions, especially for long-term monitoring.

The transducer array may be integrated into the patch by providing it with an adhesive area, which is suited for attaching the patch to a patient's body surface. Also, a textile or other flexible material may be provided with an adhesive material and used to fix the transducer array to the body surface. In particular, the patch may be configured such that it secures the transducer array in close proximity to the body surface.

The coupling between the transducer array and the control unit may comprise a wire connection, in particular an interface for data and/or power transmission between the transducer array and the control unit. In particular, the control unit and/or a part of the control unit may be arranged on the sensor array or may be integrated with the sensor array into one common part. The sensor array can be embodied especially by means of the combination of a switch (e.g. TFT) array and a transducer array.

In particular, there is a wire connection and/or a wireless interface provided, if the control unit controls the sensor array. If a wireless sensor array is implemented, the control unit may be arranged on the sensor array and/or integrated into one component with the sensor array.

In particular, if the sensor array is connected to another element by a wire, at least part of the controlling of the sensor array may be implemented on the sensor array and/or integrated into one component with the sensor array. Also, an additional control unit may be provided, e.g., in a separate PC or control unit, when there is access through wires to the sensor array. A sensor array is for example a combination of a transducer array and a TFT array. Parts of the controller can be integrated on a TFT array by an integrated circuit design or by bonding ICs to the TFT.

In particular, if the transducer is connected to another element by a wire, at least part of the controlling of the transducer array may be implemented on the transducer array and/or integrated into one component with the transducer array. Also, an additional control unit may be provided, e.g., in a separate PC or control unit, when there is access through wires to the transducer array.

In an embodiment of the system, the system further comprises a power source unit for providing energy to the transducer array and/or the control unit. The system can thus be advantageously used in a very flexible manner, in particular when a portable power source is provided. For example, the power source may comprise a battery, which is integrated in a portable device as a component of the system.

Rather than operating the transducer array as a single whole and using each element as an individual unit, a sub-array scanning architecture may be used, i.e., groups of transducer array elements are combined to functional units. In particular, neighboring elements are combined.

In particular, a control algorithm may be provided for controlling each sub-array within the transducer array. The control algorithm may control, for each sub-array, a location of the sub-array within the plurality of transducer array elements. Also, the control algorithm may control, for each sub-array, a focus depth and/or a steering angle for an ultrasound sending beam profile and/or an ultrasound receiving beam profile implemented by the sub-array of transducer array elements; such a beamforming algorithm may be implemented as a software module for a programmable unit and/or by chip design, e.g., in an ASIC.

When individual sub-arrays are controlled as functional units for sending ultrasound pulses and/or receiving ultrasound signals, the system may for example be configured to perform ultrasound based measurements of a sample by using the sub-array as a unit cell for sending and/or receiving ultrasound pulses and/or receiving ultrasound signals. In particular, ultrasound signals are detected as reflections of a previously generated ultrasound pulses, and imaging or other data processing is performed by mapping the reflected signal and its intensity to specific areas or locations.

The transducer array elements of a sub-array work as a combined "element", collecting 1D depth data sets by controlling the sending of an ultrasound pulse and collecting reflected ultrasound signals from a sample, e.g., from inside a patient's body.

Also, the system may be configured to generate 3D spatial information from 1D data sets collected by the transducer array, or one or more sub-arrays of the transducer array.

The system may be configured to determine a position for a sub-array within the transducer array such that it is not hindered by impediments for ultrasound beams, e.g., due to ribs of a patient close to the surface, due to lungs being present in the direct ultrasound transmission path or due to air bubbles between the transducer array elements of the sub-array and a sample. To collect data in order to generate, e.g., 3D spatial information, the control unit may be configured to control, for each sub-array, the location of the sub-array, as well as the focus depth and/or a steering angle for the sub-array depending on a chosen region of interest, e.g., within a patient's body.

For controlling a sub-array of transducer array elements in order to perform imaging, and, e.g., for measuring 3D spatial information, a positioning of the sub-array position may be determined and controlled within the plurality of transducer array elements. This may be carried out by controlling a signal path between a thin-film transistor (TFT) array, which is arranged to transmit control signals to the transducer array elements and/or to transmit reading signals from the transducer array elements, and the control unit of the system, in particular a mixed signal ASIC, which may be arranged on the periphery of the sensor. The control unit, in particular the switch (e.g. TFT) array, and a respective switch (e.g. TFT) driver module, may be configured to selectively switch rows and/or columns of transducer array elements to define an active sub-array and a sub-array location in a larger matrix of the system's transducer array. In particular the TFT elements contain TFT transistors, these transistors act as electronically controlled switches which can provide or interrupt an electrical connection towards a connected transducer element. The TFT driver connects to at least one but preferably multiple TFT transistors, the TFT driver provides the electronic signal to control the switching (on-off) of the attached TFT transistors.

In order to control the profile, focusing or steering of a sub-array's ultrasound wave beam a beamforming module may be used, this allows control over the direction and depth at which can be optimally measured.. The beam profile is controlled by applying delays on the individual transducer array elements or a collection of transducer array elements which provide a common input and output, i.e. a functional unit. This may be performed both for sending ultrasound pulses, thereby focusing ultrasound energy at a depth defined by a predetermined focal point and sending the ultrasound energy under a predetermined steering angle. Analog to the sending, the beam profile may be controlled for receiving ultrasound signals, e.g., in order to define a direction and a focal depth for receiving ultrasound signals, e.g., to optimally receive ultrasound signals from a predetermined location within a sample.

The complexity of the system may be defined as a characteristic in different ways. In particular, the complexity measures the number of individually driven functional units. For example, the "complexity" of the system may refer to the total amount of channels, which are used for generating ultrasound pulses and/or for receiving ultrasound signals.

The sub-array size may be chosen and configured such that a suitable signal-to-noise ratio, a specific beam profile and/or a required image quality can be achieved. For example, when a certain sub-array size SA, where SA is the product of the number of rows "n" and columns "m" (SA = n*m), is chosen, this may reduce the complexity of the system significantly.

In an embodiment, a complexity reduction module is configured to define the plurality of sub-arrays such that there is no overlap between the transducer array elements of separate sub-arrays. In other words, transducer array elements are not "shared" among different sub-arrays, i.e., attributed to several sub-arrays, but only attributed to one or none of the sub-arrays. The transducer array can therefore advantageously be controlled in a very simple and efficient manner.

In an embodiment, the system comprises at least two transducer arrays forming a least one transducer array set; and the control unit is configured to control the transducer array set as one single transducer array. By using a transducer array set for example several positions can be monitored (e.g. needed for lung monitoring) by means of one system. Further, a greater surface area can be monitored. Also, different viewpoints for monitoring a target, which can be an organ, fluid carrying structure or other volumetric feature in the body e.g. the heart and/or lung and/or chest can be established.

The transducer array units may be combined in series and/or in parallel into a single element, i.e., a single transducer array set, which is driven as a single transducer array of the ultrasound system.

Different architectures may be used to address transducer array elements individually or combined in functional units. In a row column based architecture, for example, transducer array elements that are arranged in one dimension are combined and treated as a functional unit. Such elements in one dimension may be arranged along an essentially straight line. For example, instead of controlling the single elements of a transducer array individually and/or collecting signals from all transducer array elements, all elements along one axis may be combined into a single input and/or output.. For example, all elements of one row or column are combined. Connecting a transducer element to a TFT element configured as a switch, allows to selectively connect a transducer element to the row or column, so that a sub-selection can be made of the active transducers along a single row or column. The pulsers and receivers for driving respectively reading the combined transducers array elements can each be connected to either the rows or columns of the array or a combination thereof; leading to possible configurations in the read-out electronics which we designate as row/row, column/column or row/column read-out schemes.

In an embodiment, the system is configured such that the transducer array is controlled according to a row/column based architecture. This allows advantageously to reduce the data complexity greatly. The row/column architecture may be implemented by the specific transducer array architecture, or it may be implemented by how the control unit drives the transducer array, in which case the row or column configuration may be set by the control unit.

In a row/column based architecture, the transducer array elements that are arranged along the same row or column are connected together and addressed as a single functional unit. This combination of single transducer array elements reduces the complexity from N*M (when each single transducer array element is controlled individually) to N+M, wherein N represents the number of rows and columns present in the transducer array.. Connecting a transducer element to a TFT element configured as a switch, allows to selectively connect a transducer element to the row or column, so that a sub-selection can be made of the active transducers along a single row or column.

Herein, each row and/or column unit may still consist of multiple interconnect lines forming the connections between external nodes and internal transducer array elements. Each interconnect line may herein carry a different signal, e.g., to provide a signal line, a ground line or to provide a control signal for switching the TFT transistors.

Also, an enhanced signal quality may be achieved by choosing ultrasound beam profiles with the same angle for sending ultrasound pulses and receiving ultrasound signals. To perform beam-steering for "send" and "receive" beams in the same direction, a row/row or column/column based drive & read scheme may be used. For these architectures, the "send" and "receive" channels are located along the same dimension of the transducer array, which may be configured as a 2D matrix of transducer array elements. In the row/row or column/column drive & read scheme, beam steering may be performed along one dimension. In particular, ASIC elements may be arranged along columns and/or rows of transducer array elements to allow beam-steering in two orthogonal directions. However, for each event of sending and receiving, e.g., to collect image data, only one of the two orthogonal directions may be accessed.

In particular, the elements of one row or column may be combined in different ways. For example, data of these elements as sensors may be virtually combined into one channel by a suitable control by the control unit, or the row/column architecture may be implemented by the design of the transducer array itself, e.g., by performing an integration step directly on the transducer array element level. A row/column configuration may also be implemented by a suitable chip design.

The sub-arrays may be electronically defined by a combination of control signals on the TFT array (determining which transducer element are active) and the routing of the pulsing channels and readout channels to the TFT rows or columns. For example, without limiting the invention to these features, the TFT driver, which may be part of the control unit and/or partly by the circuitry on the TFT layer, can select the active columns, while the control layer connects the read and write channels to the columns. In particular, the intersection of both may form the sub-array. In another example, the TFT driver selects the active columns, while the control unit connects the write channel on the rows and the read channels of the columns or vice versa. Also, the elements of several adjacent rows or columns may be combined in the same manner.

In an embodiment, the system is configured such that a sub-array size is provided, sub-arrays of the sub-array size are defined and the transducer array is controlled based on the individual sub-arrays. The sub-arrays can therefore advantageously be defined very precisely and adjusted to different conditions.

The value of sub-array size may be determined or given in different ways, for example in a dynamic determination step, in which an optimum sub-array size is determined depending on parameters like free computing capacity, signal-to-noise ratio or accuracy values, geometric properties of an object or of the transducer array, or calibration values, which may be determined or measured in a calibration step. In particular, the sub-array size is determined beforehand through system studies. Considerations may be, for example, the signal to noise ratio of the return signal and the ultrasound beam profile, including parameters such as beam spreading and focus beam width. Another parameter may be the sub-array size, if this is chosen too large, the sub-array would not fit in between the ribs of a patient, on whom the system is applied.

For example, a sub-array size of at least 16 rows by 16 columns may be provided. The chosen value may depend on the wavelength that is used for the ultrasound. For an array, in which the transducer array elements are spaced apart about a full wavelength, combining a larger number of transducer elements may create problems, when measuring through ribs, which have a certain distance to each other. A smaller number may be used, for example 14x14, 12x12, 10x10. For a pitch value of one half wavelength (lambda/2), the amount of rows and columns would be double, i.e., 32 rows and columns, while for a 2*lambda pitch, 8 rows and columns may be used.

In particular, the control unit may provide beam characteristics for each sub-array. Thus, the elements of this sub-array are used to generate ultrasound pulses in one specific direction and with defined pulse properties, respectively. On the other hand, the beam characteristics define ultrasound signal detection properties of the system, in particular the direction and (focus) depth where ultrasound signals are optimally detected. This is due to the fact that the focus point is "optimal" at a given depth given the best resolution.

In an embodiment, the transducer elements of each sub-array are configured to provide an analog ultrasound sensor signal and the ultrasound sensor signals of the transducer elements of each sub-array are provided to one common analog-digital converter (ADC). In particular, the individual signals of the transducer array elements of one sub-array are combined first, before they are provided to the ADC. This leads advantageously to a reduced complexity.

For example, there may be one ADC per row or per column in a row/column architecture. The ADC may be part of the data processing unit or implemented in one or more separate units. By providing the detected signals of a sub-array of transducer array elements to one ADC, only one digital signal is produced and the amount of data that is acquired is reduced.

The number of analog-digital converters that are needed by the system may be reduced from one per receive channel to one per sub-array, thus also reducing the amount of data that is generated. Consequently, significant benefits in power consumption and data rate may be reached.

Additionally, or alternatively, a software or programming implementation of sub-array beamforming may be used in the system.

Especially and explicitly disclosed is a method for ultrasound imaging of a body part of a subject comprising at least the following steps
- placing a transducer array with a plurality of transducer array elements for providing ultrasound waves on the body of the subject;
- obtaining reference ultrasound data by scanning;
- detecting a target organ and/or part of a target organ;
- reconstructing the volume of the target organ and/or part of the target organ;
- monitoring the volume of the target organ and/or part of the target organ.

In an embodiment, the step of obtaining reference ultrasound data comprises at least two different phases of scanning, wherein the at least two different phases of scanning differ in terms of resolution.

In a further embodiment, the method further comprises the step of detecting at least one artifact (such as an air bubble between the transducer array and the body of the subject, a bone or cartilage).

In a further embodiment, reconstructing the volume of the target organ and/or part of the target organ comprises scanning at least partially the volume of the target organ and/or blood vessel without producing an image.

In particular, the scanning may be based on ultrasound based pulse-echo measurements with collimated/focused and or steered ultrasound beams, wherein the ultrasound beam profiles are controlled by selecting a subset of transducer array elements of the transducer array as a sub-array within the transducer array elements. Driving or reading the transducer elements in the sub-array with different phases allows to control the beam profile.

In particular, data related to a single pulse-echo measurement provides the information of the location of the volume and the interfaces of the feature of interest e.g. a target organ and/or part of the target organ and/or vessel and/or part of the vessel. By shooting multiple of these beams over random or carefully selected locations over the volume and/or the boundaries of the feature of interest, the volume can be sparsely sampled, and this sparse data can be used to reconstruct the volume.

As described above, to reconstruct the volume, another method could use the sparse measurement data and determine the deformation against a more detailed reference volume which would have been collected in an earlier measurement, i.e. it would track the deformation of the reference volume over time with sparse measurements. Yet another method could use the imaging data directly to track the deformation directly against a reference image.

In another embodiment the reference volume can be measured in initial scanning step, which scans the volume in more detail. This scanning step can also be split up in a first detection step which takes a coarse image of the region of interest, this coarse image is then used to find/detect the volume of interest. A second, more detailed scanning step could then focus on the detected volume of interest. This advantageously reduces the amount of measurements needed for establishing the detailed reference volume.

In particular, the sparse data may be interpolated by b-spline interpolation or interpolation by fitting to a predefined shape.

Further, the method may further comprise the step of determining cardiac cycle phases including at least one of start systolic phase, end systolic phase, start diastolic phase and end diastolic phase, wherein the monitoring is performed only at one or more cardiac cycle phases.

In an embodiment, the cardiac cycle phases are determined by performing at least one ultrasound based pulse-echo measurements with at least one collimated/focused or steered ultrasound beam through a defined position of the target organ and/or part of the target organ at least two times through at least one cardiac cycle.

In particular, the method serves the purpose of operating the ultrasound system. Thus, it has the same advantages as the ultrasound system of the invention.

In general, the feature of interest may be a target organ and may comprise a heart or blood vessel and the part of the target organ may comprise a ventricle or atrium. The feature of interest may also be another structure in the body which might not be related to an organ, e.g. the volume of blood in between the organs due to internal hemorrhaging.

The method can comprise combined sparse image acquisition and tracking for data-efficient cardiac motion estimation.

It is possible that the method comprises at least the following steps:
- Performing a shape estimation on a reference frame (dense 3D image);
- Defining of sparse sampling locations, such as single beams and/or slices, for 2D image acquisition; and
- Estimating a 3D deformation which integrates the 2D motion estimated in each slice into a 3D affine motion model.

Furthermore, the method can comprise at least the following steps:
- Performing a shape estimation on a reference frame (dense 3D image);
- End-systolic temporal triggering via correlation of a small number of planes;
- Defining of sparse sampling locations, such as single beam and/or slices, for 2D image acquisition at end-systole; and
- Performing a 3D global deformation and refinement via optical flow.

Preferably, the reference frame (dense 3D image) is based on measurement results and data obtained by the system.

Also, the method can comprise reference-less automatic identification of key temporal frames in a cardiac sequence.

It is also possible that the method comprises at least the following steps:
- Estimating of pairwise correlation between all frames of an image sequence;
- Performing a cardiac cycle separation via diagonal identification; and
- Performing a local minimum estimation for end-diastole/end-systole (ED/ES) identification.

Furthermore, the reference frame estimation (for example a dense 3D image) is measured according to a two-step procedure, wherein a first step is collecting a more coarse image of the region of interest and this coarse image is used to detect the volume of interest, and wherein in a second step a detailed scanning is performed to focus on the detected volume of interest. This advantageously reduces the amount of measurements needed for establishing the detailed reference volume. Further details and advantages of the present invention shall now be disclosed in the connection with the drawings.

### It is shown in

- **Fig. 1**: an embodiment of the ultrasound system;
- **Fig. 2A-C**: detailed views of the transducer array of the embodiment of the ultrasound system;
- **Fig. 3A-D**: another embodiment of the ultrasound system;
- **Fig. 4**: another embodiment of the ultrasound system;
- **Fig. 5**: an embodiment of a method to operate the ultrasound system;
- **Fig. 6**: another embodiment of a method to operate the ultrasound system;
- **Fig. 7**: another embodiment of a method to operate the ultrasound system;

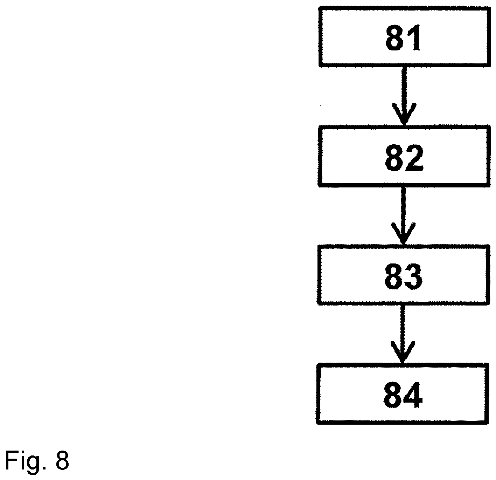

- **Fig. 8**: an embodiment of a method for correction of ultrasound systems;
- **Fig. 9**: a flowchart of the steps for a combined sparse image acquisition and tracking for data-efficient cardiac motion estimation with an ultrasound system according to an embodiment of the invention;
- **Fig. 10**: a visualization of the steps as shown in Fig. 9;
- **Fig. 11**: a flowchart and a visualization of a super sparse image acquisition and tracking for data-efficient cardiac motion estimation according to an embodiment of the invention;
- **Fig. 12**: a flowchart of the steps for a combined sparse image acquisition and tracking for data-efficient cardiac motion estimation with an ultrasound system according to an embodiment of the invention; and
- **Fig. 13**: a visualization of the steps as shown in Fig. 12.

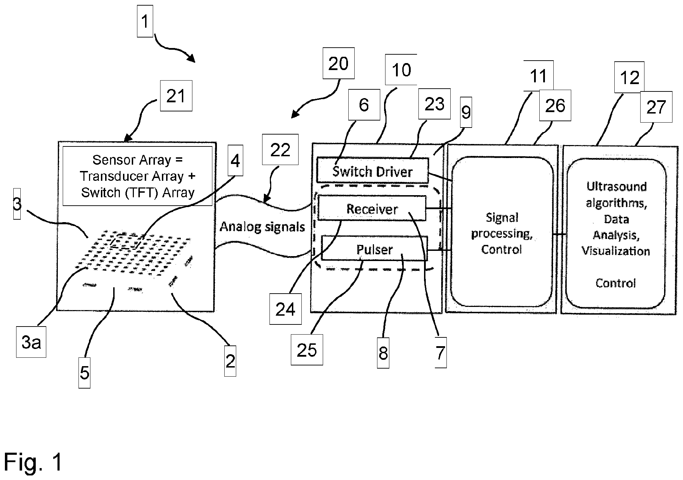

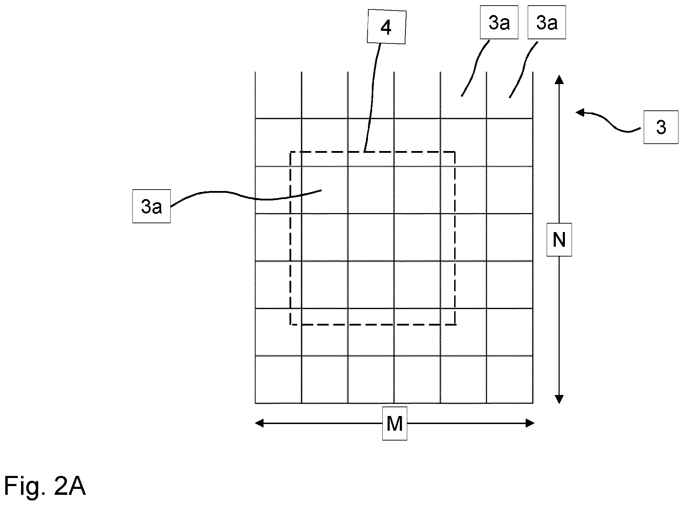

Turning to **Fig. 1** and **Fig. 2A** to **Fig. 2C****,** an embodiment of the ultrasound system is described.

**Fig. 1** shows a schematical overview and explaining the functional blocks of the ultrasound system 1.

The ultrasound system 1 according to the embodiment is configured as a wearable ultrasound system 1, in particular for long-term monitoring of a patient's cardiac activity.

In particular, the ultrasound system can be in general an ultrasound system as disclosed by WO 2019/162485 A1. The below additional features can be further realized in this already disclosed ultrasound system of WO 2019/162485 A1.

The ultrasound sensor and support electronics can be integrated in a flexible body worn format, as it is shown in this embodiment.

In the embodiment as shown in **Fig. 1****,** the ultrasound system is (at least partially) carried in a patch 2.

The ultrasound system 1 comprises a transducer array 3, which is configured as a piezoelectric micromachined ultrasonic transducer (pMUT) array 3 in the present embodiment.

The transducer array 3 comprises a plurality of transducer array elements 3a.

In the present embodiment pMUT elements 3a.

A subset of the transducer array elements 3a forms a sub-array 4.

In particular, each transducer array element 3a can consist of one or several sub-elements 3b, in particular pMUT sub-elements 3b or components, which are driven as a single larger transducer array "element" 3a.

In other embodiments, capacitive micro-machined ultrasonic transducer (cMUT) components can be used.

Other configurations with different numbers of transducer sub-elements 3b or a different arrangement of the sub-elements 3b are possible as well.

Furthermore, there is a switch array or switch module 5, which can more specific be embodied as a TFT array.

A further part of the ultrasound system 1 is the Analog Front-End 10 with the Switch Driver 6, the Receiver 7 and the Pulser 8.

The Switch Driver 6 can be embodied as a TFT driver module.

The Receiver 7 can be embodied as analog-digital converter (ADC).

The Pulser 8 can be a pulser module.

There can be a digital application-specific integrated circuit (ASIC) 9.

Further, there is an interface module 11 and an external unit 12/back-end unit 12.

A transducer array 21, which comprises a sensor array (e.g., like the transducer array 3 described above) and a switch/TFT array, is coupled with a unit implementing a switch driver 23, a receiver 24 and a pulser unit 25.

These units are configured to communicate via analog signals 22 with the transducer array 21.

In the embodiment, they are implemented in application-specific integrated circuits. Such ASICs can be located on the periphery of the sensor or on the Analog Front-End 10.

Also, they are used to receive and treat the signals, and communicate with a signal processing/control unit 26, which is implemented in an embedded compute system, e.g. on the Analog Front-End 10 with CPU, FPGA with memory or other units, these control and signal processing algorithms might also be implemented partly or completely in an ASIC.

Further processing of provided data is performed by a control module 27, incorporating for example ultrasound image reconstruction algorithms, data analysis, visualization and control algorithms.

In the case of a system 20 wired to an external unit 12, such as a PC, at least a part of the treatment algorithms can optionally be run on the embedded compute system and/or on the external unit 12. In a wireless case, such computational steps can be carried out in the embedded compute system or potentially at least partly in a holder.

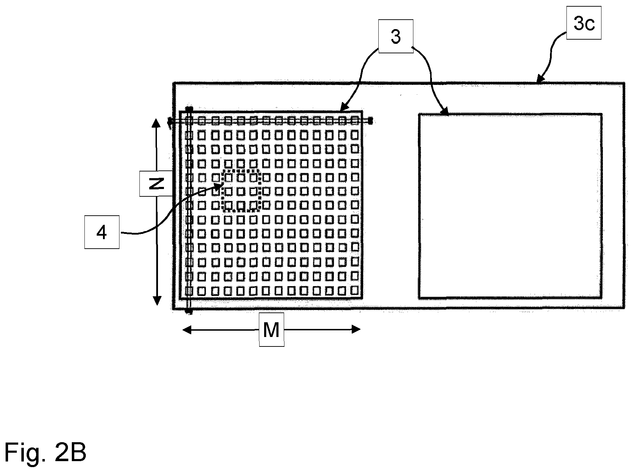

Furthermore, **Fig. 2B** shows another embodiment, wherein two or more transducer arrays 3 can form a transducer array set 3c, which is then controlled as one functional unit, i.e. "virtual" single transducer array.

In the embodiment of **Fig. 2B****,** a first and a second transducer array 3 are comprised by one transducer array set 3c. In this embodiment, the transducer array set 3c is controlled as "virtually" one single transducer array 3.

Also, **Fig. 2B** shows that the transducer array 3 comprises a plurality of transducer array elements 3a, which are arranged in a matrix with a number of N rows and M columns.

Furthermore, **Fig. 2B** shows that a subset of the transducer array elements 3a make up a sub-array 4, which is herein controlled as one unit. The sub-array 4 has a number of n rows and m columns.

**Fig. 2B** also shows that elements 3a along a row and/or a column of the transducer array 3 are interconnected with one or more interconnecting lines, a so called row/column based architecture. The elements 3a connect in parallel to the interconnects, with a switch, in particular a TFT switch, determining if an element 3a is connected or not and to which signal path it connects. Thus, rows and/or columns of the transducer array 3 can be controlled together. This allows simplifying the system by switching rows and/or columns of elements 3a together.

The transducer array 3 of the embodiment is flexible. Here, the transducer array elements 3a are arranged on a flexible substrate, e.g., comprising a flexible polymer material.

Furthermore, the ultrasound system 1 comprises a switch array which can be embodied as a thin-film transistor (TFT) array 5, comprising a plurality of TFT elements.

The switch array 5 is arranged such that its switch elements (or TFT elements when embodied as a TFT array) control corresponding pMUT elements 3a. In particular, each TFT element is assigned to one pMUT element 3a.

In the present embodiment, a TFT array 5 with 150 * 150 TFT elements is used, which are arranged in a 2D matrix with perpendicular rows and columns. The array size depends in particular on the size of the respective pMUT array 3, and it may differ in different embodiments.

Similar to the transducer array 3, the TFT array 5 of the embodiment is also flexible and/or stretchable. Here, the TFT array elements are arranged on a flexible substrate, e.g., comprising a flexible polymer material.

In particular, the TFT array 5 is arranged on the same substrate as the transducer array 3.

In particular, the integration may be monolithic or heterogeneous. In the present exemplary embodiment, the sensor layer and TFT layer may be fabricated separately and joined in a wafer-to-wafer bonding process, followed by the processing of an interconnect module.

In particular, "Heterogeneous Integration (HI)" may refer to the assembly and packaging of multiple separately manufactured components onto a single chip in order to improve functionality and enhance operating characteristics. In the present embodiment, one single array is created with a single functionality (sensing), implying a monolithic device.

In particular, attention is drawn to **Fig. 2B****,** wherein an example of a row/column architecture is demonstrated.

In the embodiment, both the pMUT array 3 and the TFT array 5 are integrated into an adhesive patch 2.

In the embodiment, the adhesive patch 2 has a size of about 10x10 cm. The size may vary in different embodiments.

The adhesive patch 2 comprises a material that is suitable for the purpose of attaching the ultrasound system 1 and in particular the transducer array 3 to a patient's skin. Therefore, biocompatible and breathable materials are used. The adhesive layer on the patch is used to attach the patch to the body and to make sure that the position of the transducer array 3 is essentially constant with respect to the patient's skin.

In another embodiment, an "acoustic matching layer" is provided in between the skin and the transducer array 3.

The system 1 also comprises a functional block for the analog control of the electronics, a so called Analog Front-End.

The Analog Front-End 10 is coupled with the transducer array 3 and the TFT array 5 for providing options such as control and read-out for the transducer array 3 and/or the TFT array 5. To this end, both data and electrical power may be transferred over the coupling between the Analog Front-End 10 and the patch 2 with transducer array 3 and TFT array 5.

According to the embodiment, the Analog Front-End can be implemented in an application specific IC (ASIC) or as circuits on the TFT layer or as discrete components or a combination thereof. All the physical components of which the analog front-end is comprised can be either integrated directly on the TFT as integrated components, on the periphery of the TFT as IC's or on a PCB (flex or rigid) physically attached to the sensor 2 through physical interconnects.

In different embodiments, the application specific IC's may be either integrated on the periphery of the TFT layer or on the PCB.

Here, a TFT driver module 6 is provided on the Analog Front End 10, which is configured to provide a low-level interface to control and manage the TFT array 5 and its individual TFT elements, respectively. In an embodiment, a gate driver is integrated with the ASIC on the periphery of the TFT layer. In another embodiment the driver circuitry could be designed partly or completely in the TFT layer.

In particular, the TFT driver module 6 of the present embodiment can be integrated together with send and receive modules for controlling the generation of ultrasound pulses and detecting ultrasound signals, respectively. All these functional modules form part of the analog front-end.

Also, a receiver 7 is provided as part of the analog-front end 10, which is configured to convert an analog input signal to a digital output signal.

Also, a pulser module 8 is provided as part of the analog front end 10, which is configured to control the transducer array 3 such that ultrasound pulses are generated. In particular, a beam-forming is performed here. Delay values may be set individually for each pulser unit of the system 1 that generates an ultrasound pulse. The delays on the different channels, in particular each pMUT element 3a, of the pulser unit will determine the focusing and the beam profile.

Also, a digital middle-end is provided , which is configured to perform part of the control operations for the ultrasound system 1, and specifically to control the activity of the transducer array 3 and the TFT array 5 for generating ultrasound pulses and detecting ultrasound signals. The digital middle-end is also configured to perform signal processing on the signals received from the analog front-end.

As part of the digital middle end, microprocessors may be provided and/or other units for performing operations for controlling the ultrasound system 1 and its electronic components.

Thus, the ultrasound system 1 of the embodiment provides an easily customized ultrasound sensor and monitoring device.

The interface module provides the possibility to build up a connection based on a wire connection and/or a wireless connection. In the present embodiment, examples for connection methods include USB and Ethernet as well as via Bluetooth and WiFi networks.

The interface module is here configured to allow for data communication and/or power connections with external units.

In the embodiment, the PCB with its components and the TFT array 5 constitute an integrated control unit and data processing unit. PCB and TFT array 5, and the transducer array 3, may in different embodiments, be configured as units that are formed separately or they may be incorporated in a common unit.

In a further embodiment, a remote or cloud based back-end unit 12 may be accessed through the interface module, either directly or indirectly via another device, such as a computer that is connected to a local or wide area network. The back-end unit may perform operations such as to reading and storing data, data analysis, configuration of the device, providing further information and administrating access the acquired data based on an authentication system.

Turning to **Fig. 2A****,** a detailed view of the transducer array 3 of the embodiment of the ultrasound system 1 is described.

The transducer array 3 has a matrix of pMUT elements 3a, which are only schematically shown in a small number.

The system 1 is configured such that a sub-array 4 of pMUT elements 3a may be defined. The elements 3a of a sub-array 4 are treated as one functional unit for generating ultrasound pulses and/or detecting ultrasound signals.

In the embodiment, an ASIC performs steps for defining sub-arrays 4 within the transducer array 3. The definition of sub-arrays 4 may be carried out in different ways, e.g., by way of the chip design or by other units controlling the transducer array 3.

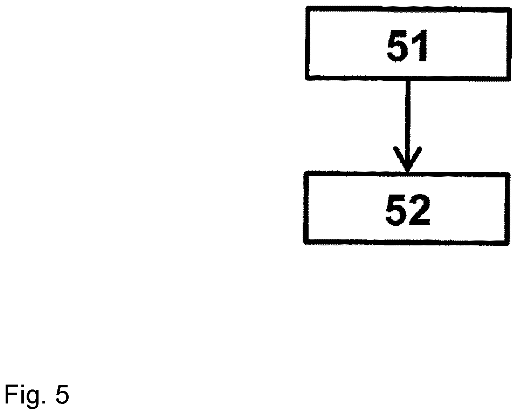

In the present embodiment, the transducer array 3 may comprise N selected out of the range 40 to 250 rows, especially 75, 100, 124 or 150 rows and M selected out of the range 40 to 250 columns, especially 75, 100, 124 or 150 columns, which are ordered in a two-dimensional matrix. On the other hand, a sub-array size value may comprise 8 to 32 especially 16 rows by 8 to 32 especially 16 columns is defined. Thus, groups of 162 elements 3a are grouped into one sub-array 4. This leads to a considerable reduction in system complexity and data volume, respectively. This aspect is further described below with reference to **Fig. 5****.**

Herein, the interrelated elements 3a are chose such that they are neighboring to each other.

In further embodiments, this definition of sub-arrays 4 may be configured in different ways, such as, in a configuration step, by inputting a predetermined sub-array size value, which defines the number of pMUT elements 3a to be included in a sub-array 4, and/or by inputting a location of a sub-array 4 within the pMUT array 3. Also, the form of a sub-array 4, i.e., its dimensions size, may be determined by an input value.

In one embodiment, the sub-array size may be defined during the system development. In the products itself, the sub-array size can be fixed, without an option to dynamically reconfigure this value; in this case, a fixed size would be used.

Said values to control how sub-arrays 4 are defined may also be determined by the digital Middle End and/or digital back-end, part of this functionality might be implemented in an ASIC or another control element of the ultrasound system 1, or by an input over the interface module, in particular depending on an algorithm. Such an algorithm for determining parameters for defining the sub-arrays 4 may use input values such as data/image quality requirements, a signal-to-noise ratio, information about factors interfering with the operation of the system, user input and configuration information, and/or other parameters.

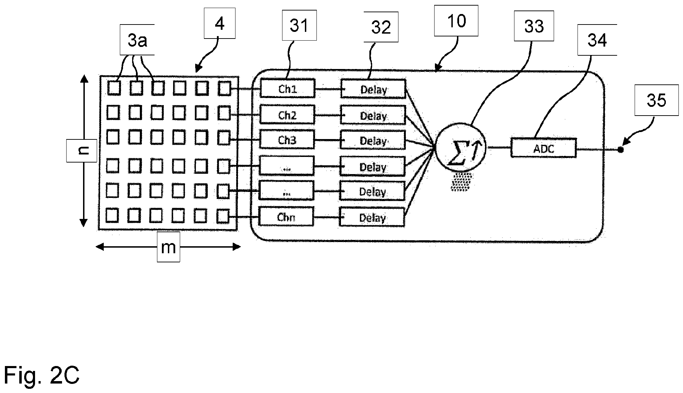

Turning to **Fig. 2C****,** an embodiment for implementing a row/column based micro beam-former is schematically described.

A sub-array 4 has a number of n row and m columns, and is connected to a micro beam-former 30. In this specific case, the n rows of transducer array elements 3a are combined into one output channel 31 each. These output channels are named "Ch1" to "Chn".

For the transducer array element 3a of the sub-array 4, the micro beam-former 30 combines the n analog input channels 31 from the sub-array 4 into one single digital output channel 35.

To this end, a delay operation is performed by delay units 32 and a sum unit 32 is subsequently used to coherently add the n analog inputs together.

An analog-digital converter 34 is used to convert the resulting analog signal to a digital signal, which is output through a digital output channel 35.

In further embodiments, each channel 31 can do some analog processing of the input signals before its signal is combined in the analog beam-former 30.

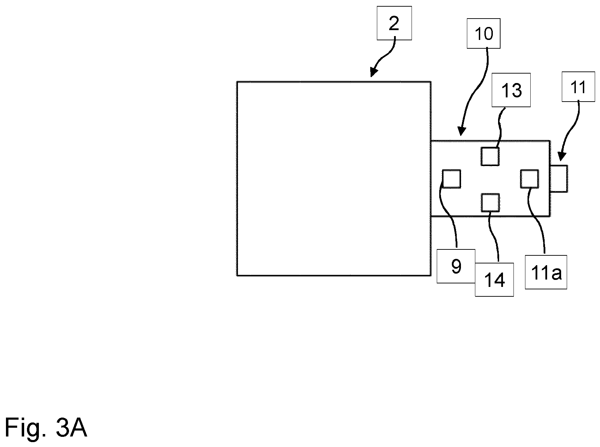

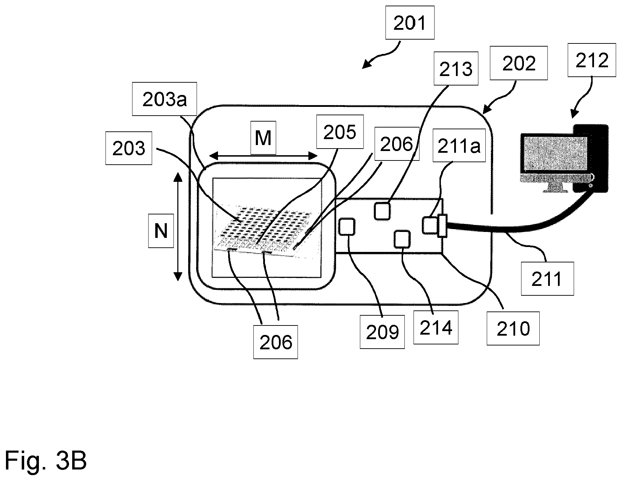

Turning to **Fig. 3A** to **Fig. 3E**, other embodiments of the ultrasound system are described. Reference is made to the embodiments of the ultrasound system as described above. Figures 1 and 3A to 3D are meant to present analog embodiments of the system, although different aspects are highlighted and different elements are explicitly shown.

In **Fig. 3A****,** a PCB 10 is shown connected to the patch 2. The transducer array 3 and the TFT array 5 are not shown, since they are also integrated with the patch 2. Also, the interface module 11 is directly connected to the PCB 10.

On the PCB 10, circuits for controlling the system 1 are shown.

The ASIC 9 is provided on the PCB 10.

Also, an interface control module 11a is provided and implemented with another IC. It provides a low-level interface to the interface module 11, thereby allowing to control the interface module 11.

Also, a memory module 13 is provided, herein specifically configured as a random access memory (RAM) module 13.

Also, a power management module 14 is provided.

In another embodiment, a power source is provided, for example a battery as further explained below with reference to **Fig. 3E.** In this case, access to the stored power is provided to the system components via the power management module 14. In this case, the interface module 11 may be configured to provide a wireless data connection, in particular to a control unit.

In the present embodiment, another way of providing power to the system may be used, such as a connection to an external power source or an external power grid.

The ASIC 9, interface module ASIC 11a, the memory module 13 and the power management module 14 may be implemented in different ways, some of which may be known in the art. For example, a microprocessor, an ASIC chip and/or other units may be used.

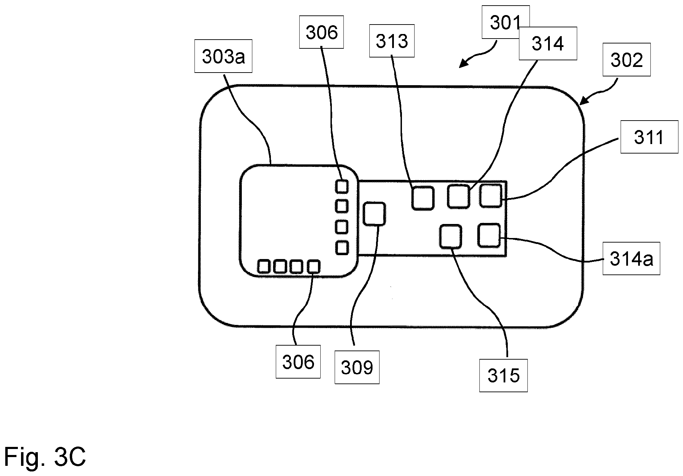

Turning to **Fig. 3C****,** another view of an embodiment equivalent to the one of **Fig. 3A** is described.

In this embodiment, a system 201 comprises a unit 203a, in which a transducer array 203 and a TFT array 205 are integrated. A flexible PCB 210 is coupled to this unit 203a, and both are integrated in a patch case 202.

The PCB 210 might be rigid, flexible, stretchable or a hybrid combination thereof.

The system 201 contains a module 206 with an ASIC with 16 channels, containing an analog front-end functional block and optionally a complete or partial digital middle/back end. The ASIC module 206 can be integrated either on the sensor unit 203a or on the PCB 210.

In particular, the flexible PCB 210 is configured such that it is flexible on the top of the sensor, in particular on top of the sensor unit 203a. The physical link between the PCB 210 and the sensor unit 203a being made by interconnects such as in particular flexible interconnects.

The flexible PCB 210 comprises components for an embedded computer system, such as a control device 209, comprising for example CPU, FPGA and/or other elements. The flexible PCB 210 further comprises a physical interface module such as e.g. a USB connector 211 and a USB transceiver 211a, by which the system 201 is coupled to a PC 212.

The flexible PCB 210 also comprises a RAM module 213 and a power management IC 214.

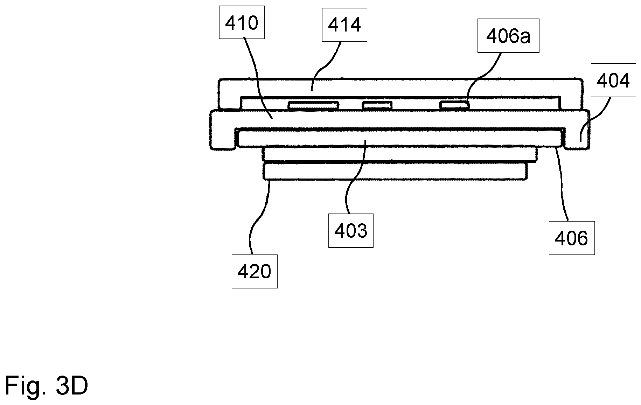

Turning to **Fig. 3D****,** another view of an embodiment equivalent to the one of **Fig. 3A** is described.

A system 301 comprises a flexible packaging, in particular a patch case 302, into which a sensor unit 303a and a flexible PCB 310 are integrated. The flexible PCB 310 is coupled to the sensor unit 303a.

The PCB 310 might be rigid, flexible, stretchable or a hybrid combination thereof.

The system 301 contains a module 306 with an ASIC with 16 inputs, containing an analog front-end functional block and optionally a complete or partial digital middle/back end. The ASIC module 306 can be integrated either on the sensor unit 303a or on the PCB 310.

The flexible PCB 310 has a control device 309, a wireless transceiver 311, a memory module 313, a battery gauge 314, a wireless/wired charger 314a and a power IC 315 integrated on it.

In particular, the flexible PCB 310 is configured such that it is flexible on the top of the sensor, in particular on top of the sensor unit 303a. The physical link between the PCB 310 and the sensor unit 303a being made by interconnects such as in particular flexible interconnects.

Also, a flexible battery may be provided.

Turning to **Fig. 3E,** a schematic cross-section of an embodiment, such as the embodiment of **Fig. 3D****,** is described.

The system 401 comprises a sensor 403.

An acoustic matching and/or glue layer 420 is applied on one side onto the sensor 403, to enable good contact to a subject's skin.

On the other side of the sensor 403, a periphery 406 section is arranged. In particular, electronics for controlling the sensor 403 are arranged in the periphery 406.

As a layer above the periphery 406, interconnects 404 are arranged. In particular, the interconnects 404 can connect elements of the sensor 403, e.g., in a row/column architecture.

In a layer above the interconnects 404, IC elements 406a are arranged.

On top of the above-described arrangement, a flexible battery 414 is arranged. This flexible battery 414 might also be arranged next to IC elements 406a and/or the PCB 410.

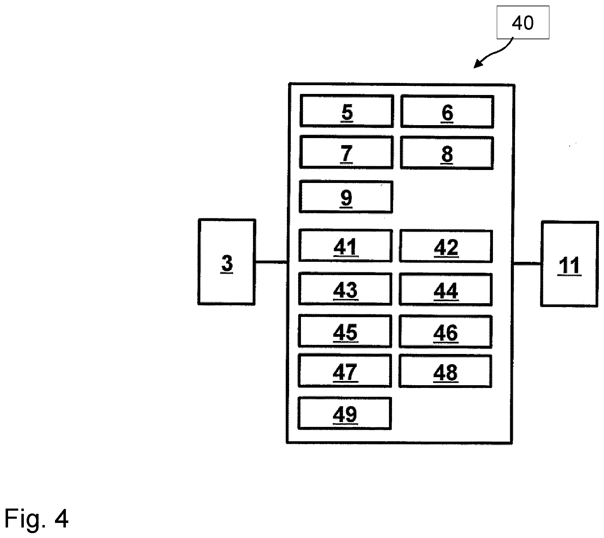

Turning to **Fig. 4****,** a schematic view of another embodiment is shown. The shown components may also be present in the embodiments described above; similar or equivalent elements have the same reference numerals and are not described again in detail.

Some or all of the elements and modules shown in **Fig. 4** may be implemented in different units, such as separately formed devices, or they may be implemented by way of program modules within the same computational unit and/or a digital memory device.

The combination of elements and modules of this embodiment is to be understood as exemplary, and as comprising a large number of optional features, which are not necessarily linked to each other or to be combined in one embodiment of the invention.

In **Fig. 4****,** the transducer array 3 is shown, which is linked to a control unit 40 (controlling the transducer array 3), which may be configured at least partially on a PCB 10.

The connection between the transducer array 3 and the control unit 40 is a direct and cable-bound connection. However, in an alternative embodiment, an indirect and/or wireless connection is possible.

For instance, the control unit 40 can be attached to the patch 2 (possible for wireless patches and wired patches). Further, there can be an additional control unit in a separate computer, in particular for the wired version.

The transducer array 3, configured for providing ultrasound waves and/or receiving ultrasound signals, comprises a plurality of transducer array elements 3a.

The control unit 40 comprises the TFT array 5, which was already described above.

The control unit 40 further comprises a dedicated data processing unit 41, which may in different embodiments comprise some of the other modules (as specified below with reference numerals 41 to 49) or which may in different embodiments be implemented by several individual modules. The data processing unit 41 receives and processes data from the transducer array 3.

Moreover, the control unit 40 comprises a reference module 45.

Also, the control unit 40 comprises a volume reconstruction module 46, in particular for reconstructing the volume of a target organ or part of a target organ which is in this embodiment the left ventricle of a subject equipped with the ultrasound system 1.

Furthermore, the control unit 40 comprises a selected monitoring unit 47, in particular for monitoring the left ventricle.

Further, the control unit 40 comprises a correction module 44.

Also, the control unit 40 comprises a complexity reduction module 42.

Additionally, the control unit 40 comprises a deformation correction module 43.

In addition to that, the control unit 40 comprises an artifact determination module 48.

Furthermore, the control unit 40 comprises a phase determination module 49.

The system 1 according to the embodiment is controlled by integrated support electronics. The support electronics comprise mixed signal ASIC elements distributed along the periphery of the sensor. The sensor may comprise the transducer array 3. The ASIC elements could also be located on the PCB 10.

Also, the rest of the electronics is integrated on the flexible PCB 10, which is in turn connected to the sensor.

The ASIC elements are distributed along the edge of the flexible ultrasound sensor and have some or all of the following functions:
- Actuating the mechanical transducers, i.e., the transducer array elements 3a;
- Processing and digitizing signals received by the transducer array elements 3a;
- Controlling the switching of the TFT array 5; and/or
- Dynamically reconnecting the analog channels, i.e., from different transducer array elements 3a, to different sets of columns/rows.

The flexible PCB 10 contains a digital "master" ASIC 9 and/or a microprocessor, for performing digital signal processing, and control of the other ASIC elements. The digital signal processing and/or control may also be implemented in part or completely on the mixed signal ASICs distributed along the periphery and/or edge of the sensor (e.g. routed to the backside of the sensor on a PCB by means of interconnects).

By performing row/column addressing of the pMUT elements 3a of the transducer array 3, the complexity of the system 1 is reduced from N*M to N+M, wherein N and M represents the amount of rows and columns in the array. In case of a fully wired array (i.e., when each transducer array element 3a is controlled individually) each element 3a can be addressed individually (hence the N*M complexity), while for a row/column based architecture the elements 3a along the same row or column are connected together and addressed as a single unit, reducing the complexity to N+M. Each unit could still consist of multiple interconnect lines forming the connections between the external nodes and the internal array elements. Each interconnect line can carry a different signal, e.g. to provide a signal line, a ground line or to provide a control signal for switching the TFT transistors.

A hardware beam-former may be used to combine, e.g., 16 analog channels, which connects each to an interconnected unit of transducer array elements 3a, to a single ADC 7, i.e., one digital channel. The number of combined elements 3a determines the factor by which the system complexity is reduced. In particular, a plurality of ADCs 7 may be provided and these may be linked to a defined sub-array 4 of transducer array elements 3a.

In the embodiment, a sub-array 4 is used as one functional unit, e.g., a "single entity" for scanning the full array (a unit cell). This single entity is electronically controlled and/or scanned over the larger transducer array 3. In particular, the sub-arrays 4 are electronically "shifted" per single row/column of the transducer array 3.

In the present embodiment, a sub-array 4 is electronically controlled as a single unit. In particular, there is one ASIC for each sub-array 4.

In further embodiments, multiple sub-arrays 4 may be provided for each ASIC. The number is determined by the accessible power, cost restrictions, acceptable compactness and/or speed requirements for the system.

By using several ASICs for controlling individual sub-arrays or controlling multiple sub-arrays per ASIC, faster scanning is possible, since several measurements, in particular "single entities" or "functional units", are operated in parallel.

In the following, aspects and methods of operating an embodiment as described above are described. In particular, the following description is based on an embodiment similar to the one of **Fig. 4****.**

When the transducer array 3 is placed on the body (thorax) of the subject, it does not have any prior information on the features of the body on which it is placed. Thus, the reference module 45 provides a detailed scanning of the region of the total transducer array 3 (matrix of transducers). This scanning will allow to detect the left ventricle as feature of interest/target. Any method known in the art can be used for feature detection, e.g., correlation with known feature, neural networks etc. Furthermore, this scanning allows to detect incorrect placement of the transducer array 3 on the thorax of the subject, and/or it may be evaluated, whether the left ventricle is only partially visible from the position of the transducer array 3.

Based on the reference ultrasound data obtained by the reference module 45, the volume reconstruction module 46 reconstructs the volume of the left ventricle by providing a collection of collimated ultrasound beams by a sub-array 3a of the transducer array 3.

Thus, the volume reconstruction module 46 can control a location of a sub-array 4 within the plurality of transducer array elements 3a. Also, the volume reconstruction module 46 can control, for each sub-array 4, a focus depth and/or a steering angle for an ultrasound sending beam profile and/or an ultrasound receiving beam profile implemented by the sub-array 4 of transducer array elements 3a; such a beam-forming algorithm may be implemented as a software module for a programmable unit and/or by chip design, e.g., in an ASIC.

Another method could use different sparse pulse-echo measurements of the left ventricle, and use these measurements to determine the deformation against a more detailed reference volume, which would have been collected in an earlier measurement, i.e., it would track the deformation of the reference volume over time using only sparse measurements. Yet another method could use the imaging data directly to track the deformation directly against a reference image.

The artifact determination module 48 can be used for identification of artifacts and avoiding obstructions to ultrasound imaging caused by artifacts, such as air bubbles between the transducer array and the skin of the patient equipped with the system or bones, e.g. ribs. In particular, strong ultrasound reflection of the ribs may be used for identification. The artifact determination module 46 in general may combine groups of transducer array elements 3a into a sub-array 4.

Combining of groups of transducer elements 3a into a sub-array 4 can alternatively be done by the complexity reduction module 42.

Also, the complexity reduction module 42 may be implemented as a software module, comprising instructions for computational operations that implement the function of complexity reduction. Such a software module may in particular be implemented together with the control unit 40.

Specifically, the complexity reduction module 42 may be comprised, at least partially, by the control unit 40 and/or by the data processing unit 41.

Further, the selected monitoring unit 47 is configured to monitor the left ventricle (by means of the sub-array 4).

In particular, the computational load may be further reduced by only performing a detailed scanning of the volume at discrete time instances of the cardiac cycle:
The phase determination module 49 is configured for determining cardiac cycle phases in particular end-systolic phase and end-diastolic phase, as for evaluation of the stroke volume only the end-diastolic and end-systolic phases are of importance. For this a single (alternatively a few) ultrasound pulse-echo measurement(s) is/are done through a carefully chosen position of the left ventricle or other reference, from which the cardiac cycle can be determined. This is done several times through the cardiac cycle so that a good graph can be obtained of the contraction over time, e.g., 40 to 200 "samples" could be taken over the full cardiac cycle. From these sampled curves, the end-diastolic and end-systolic phases are detected by analyzing the derivative to determine min/max in the curve(s). Alternatively, several cycles can be measured, which allows for accurate estimation of the end-systolic/diastolic phases. The regularity of the heart rhythm then allows to determine the next instance of the end-diastolic/end-systolic phases. Another option may be to train a neural network, which takes as input the time series and gives as an output the end-diastolic and systolic phases. Yet another option would be to identify focus on a feature of the heart which provides a good indication of the heart cycle, such as e.g. a valve of a heart chamber. The phase can also be determined by monitoring the heart cycle with other signals like e.g. an ECG signal.

Therefore, a detailed volume scanning at the end-diastolic and end-systolic phase is enabled by means of the selected monitoring unit 47.

For instance, once the end-diastolic and systolic phases have been detected by the phase determination module 49, the system 1 can switch from the phase determination module 49, and send a single "scanning" beam to the volume reconstruction module 46 and send a sufficient amount of beams to reconstruct the volume.

For data treatment, in particular for monitoring and/or clinical applications, a cardiac output variation over certain time periods, e.g., over a day, may be determined by the system 1. Also, motion data may be provided and, e.g., linked to measured cardiac activity, such as heart rate and other parameters. For example, an accelerometer may be integrated or connected to the system 1. Also, a movement of the patient's thorax may be determined with an accelerometer. Furthermore, the influence of breathing and physical activity may be considered. A movement of the patient may be measured, e.g., a rhythm of breathing. Furthermore, the lungs may be monitored by the ultrasound system as well.

Also, changing properties of the transducer array 3, e.g., a pMUT array 3, may be analyzed in order to obtain information on the deformation of the transducer array 3.

Also, an automatic evaluation may be carried out to determine whether the signal-to-noise ratio is too low, i.e., below a predetermined limit, and the reliability of measurements is affected.

In particular, ultrasound based pulse-echo measurements using focused beams may be used to sample a probe volume.

As a way to reduce the volume of acquired data, which needs to be processed and analyzed during monitoring of cardiac activity, sampling may be limited to end-diastolic and/or end-systolic volumes of a heart chamber of or another volume.

In order to track a heart rhythm, a single reference beam may be used to track heart motion and evaluate end-diastolic and/or systolic phases.

To make sure that the monitoring provides most reliable data, different measures may be taken: Ultrasound pulse-echo measurements may be used together with a sampling of selected, reduced measurements of few spatial data points. Also, ultrasound pulse-echo measurements may be used together with a varying pulse repetition frequency, wherein the pulse repetition frequency is reduced outside of defined measurement windows. The pulse repetition frequency may relate to a regular interval; also, pulse-echo measurements may be limited to well-defined time windows. For example, a higher pulse repetition frequency may be set within these time windows, in order to gather more pulse-echo measurements and perform a denser sampling of the volume on interest, while the pulse repetition frequency is set lower outside the time windows, e.g., to track the phase of the heart contraction with lower time resolution.

The monitoring may comprise, in particular with a given frequency, steps of calculating a volume, calculating the volume of ellipse in order to approximate a volume and/or volume change, segmentation and integration of image data, detecting a base line value, calibrating the system and/or monitoring strain, in particular on the transducer array, in order to estimate deformation and/or a dynamic form factor of the transducer array.

In particular, monitoring and calibration are implemented as two distinct steps in the operation of the system, in particular where the transducer array is included in a flexible patch:
a) The patch is applied to a patient's body.
b) The patch would perform a calibration step (detecting its deformed shape due to the shape of the thorax).
c) The system would perform monitoring and tracking the volume of the heart over time.

During monitoring, potentially one would need to correct for dynamic shape changes of the patch, e.g., due to breathing or movement of the patient. This could be done by, e.g., a) performing a recalibration at regular intervals, b) measuring deformation with strain monitoring, c) using a tracking algorithm for the shape deformation.

The monitoring of the volume could be done in different manners:
a) Establish a detailed baseline shape of the ventricle, and track the changes in this baseline shape over time with limited number of ultrasound based pulse-echo measurements.
b) Measure with a limited number of ultrasound based pulse-echo measurements and establish directly the volume from these.

In particular, image based metrics may be used for the calibration step. This may enable a deformation-free reconstruction of images even from a deformed transducer array, i.e., without reconstruction of the concrete form factor of the transducer array. E.g., wrong shapes may be assumed and true images may be reconstructed based on these wrong shapes.

Also, shape estimation for the transducer array may be performed based on deformation-free reconstruction data.

Measurements may be carried out by generating reference beams towards a diffuse reflector, and detecting the reflected ultrasound signals with another known sub-array 4, in order to evaluate displacement of the sub-arrays 4 relatively to each other, which would allow for estimating the deformed shape of the array 3.

Also, changing properties of the transducer array 3, e.g., a pMUT array 3, may be analyzed in order to obtain information on the deformation of the transducer array 3.

Different detection methods may be used, in particular for monitoring cardiac activity in a patient: A left ventricle may be detected and targeted for measuring, in particular measuring its volume and the dynamic change in volume.

Moreover, ribs and other obstructions in the patient's body may be detected in order to reduce noise in the measurements and to optimize the choice of a suitable ultrasound beam-forming and focusing. In particular, strong ultrasound reflection of the ribs may be used for identification.

In order to define a sub-array 4 of the transducer array 3, several steps may be provided and carried out, individually or in combination with each other. A positioning step may be carried out for sub-arrays 4 in order to control their position within the transducer array 3, i.e., for choosing suitable elements 3a and assigning them to the sub-array 4. Also, a sub-array's position may be shifted, in particular randomly, to avoid long term stress on specific elements 3a, leading to degradation of the elements, in particular pMUT elements 3a.

The use of ASIC elements may improve the security of the device, since the respective control methods are not easily manipulated, at least not without direct physical access to the device.

In one embodiment, the ultrasound system 1, in particular for cardiac monitoring, comprises a transducer array 3 with a plurality of transducer array elements 3a, a control unit 40, which is coupled to the transducer array 3, for controlling the transducer array 3, and a data processing unit 41 for receiving data from the transducer array 3 and processing the received data. Therein, the ultrasound system 1 further comprises a complexity reduction module 42, which is configured to define at least one sub-array 4 of transducer array elements 3a and to control the at least one sub-array 4 as a functional unit for sending at least one ultrasound pulse and/or receiving at least one ultrasound signal.

In one embodiment, the ultrasound system 1, in particular for cardiac monitoring, comprises a transducer array 3 with a plurality of transducer array elements 3a for providing ultrasound waves, a control unit 40, which is coupled to the transducer array 3, for controlling the transducer array 3, and a data processing unit 41 for receiving data from the transducer array 3 and processing the received data. Therein, the system further comprises at least one reference module 45 configured for obtaining reference ultrasound data, at least one volume reconstruction module 46 configured for reconstructing the volume of a target organ or part of a target organ, and at least one selected monitoring unit 47 configured for monitoring the volume of the target organ and/or part of the target organ.

In one embodiment, the ultrasound system 1, in particular for cardiac monitoring, comprises a transducer array 3 with a plurality of transducer array elements 3a, a control unit 40, which is coupled to the transducer array 3, for controlling the transducer array 3, and a data processing unit 41 for receiving data from the transducer array 3 and processing the received data. Therein, the ultrasound system 1 has at least two operating modes, namely a sleep mode and a measuring mode. The control unit 40 is configured, when the sleep mode is activated, to have a low frequency system clock running at a predetermined sleep frequency, and, when the measuring mode is activated, to set a high frequency system clock to a measuring frequency.

In one embodiment, the ultrasound system 1, in particular for cardiac monitoring, comprises a transducer array 3 with a plurality of transducer array elements 3a, wherein the transducer array 3 is flexible, a control unit 40, which is coupled to the transducer array 3, for controlling the transducer array 3, and a data processing unit 41 for receiving data from the transducer array 3 and processing the received data. Therein, the system 1 further comprises at least one correction module 44 configured for correction of effects in analog ultrasound signals caused by bending of the flexible transducer array 3.

In one embodiment, the ultrasound system 1, in particular for cardiac monitoring, comprises a transducer array 3 with a plurality of transducer array elements 3a, a control unit 40, which is coupled to the transducer array 3, for controlling the transducer array 3, and a data processing unit 41 for receiving data from the transducer array 3 and processing the received data. Therein, the ultrasound system 1 further comprises a deformation correction module 43, which is configured to perform a deformation correction step, wherein deformation information is determined for the transducer array 3.

The embodiments may be freely combined with each other. Further modules as described above, for example with reference to figure 4, may be provided optionally to allow further functionalities as described in this specification.

Turning to **Fig. 5****,** a method for operating the ultrasound system 1 is described. The following description is based on the embodiment of the ultrasound system 1 described above with reference to, e.g., **Fig. 4****,** but other embodiments of the ultrasound system 1 may be suited as well.

In this context, "complexity reduction" denotes a reduction of the amount of read and/or write channels needed to address the sensors, in particular the transducer array elements 3a or a sub-array 4.

This can be reached by
a) combining sub-elements 3b into a single functional element 3a (cf. **Fig. 2A**);
b) connecting elements 3a together in a row/column architecture, leading to a complexity reduction from N*M to (N+M) (cf. **Fig. 2B****)**;
c) driving a sub-array 4 instead of the full transducer array 3 at once, leading to a complexity reduction from (N+M) to (n+m); and/or
d) using a micro beam-former to combine the n (or m) analog input channels 31 of a sub-array 4 into a single digital input channel 35 (cf. **Fig. 2C**).

In a step 51, a plurality of sub-arrays 4 is defined within the transducer array 3. In a subsequent step 52, the individual sub-arrays 4 are controlled as functional units for sending ultrasound pulses and/or receiving ultrasound signals.

In the system of the embodiment, the steps 51, 52 are performed by the complexity reduction module 42, which is comprised by the control unit 40 of the ultrasound system 1. Herein, the complexity reduction module 42 is configured to control other units and modules, especially the TFT driver module 6, the receiver module 7, and/or the pulser module 8, depending on whether a send or a receive cycle is active.

The steps 51, 52 may be performed in a cyclic manner, i.e., they may be repeated for example at given intervals or triggered by predefined events and/or control signals.

In one embodiment, the system is configured such that the sub-array 4 is defined dynamically and may change its position.

In the present embodiment, the sub-arrays 4 are defined such that there is no overlap between the transducer array elements 3a of separate sub-arrays 4, i.e., no transducer array elements 3a are "shared" among different sub-arrays 4.

In another embodiment, a transducers array set 3c comprises at least two transducer arrays 3 and the control unit 40 is configured to control the transducer arrays 3 as one functional unit.

Also, in an embodiment, the transducer array 3 may be controlled according to a row/column based architecture.

Furthermore, a sub-array size may be predetermined or provided or determined by an algorithm, and sub-arrays 4 of the sub-array size are defined based on this value. The transducer array 3 is then operated based on these individual sub-arrays 4.

Also, the transducer elements 3a are controlled by the control unit 40, in particular via the TFT array 5. The transducer array elements 3a of each sub-array 4 detect an analog ultrasound sensor signal, and produce an analog signal. This analog signal is provided to the receiver module 7.

In particular, analog signals from the transducer array elements 3b of one sub-array 4 are processed by one common receiver module of the system 1. In further embodiments, different receiver modules 7 may be provided, e.g., in order to speed up data acquisition and processing.

Furthermore, a micro beam-forming architecture can be implemented by a hardware beam-forming module 43, such as a specialized ASIC element and/or another element of the control unit of the system 1.

Also, data may be filtered after it has been received from the transducer array 3. This may be carried out by a filter module 44, which may act on analog and/or digital signals. Depending on which filter is applied, filtered data is obtained with a reduced spatial and/or temporal resolution.

In another embodiment, a filter can be configured by adjusting filter properties. Thus, filters may be activated and deactivated, the impact of a filter on the data may be adjusted and/or a resolution of the filtered data may be adapted, e.g., to the needs for monitoring specific organs and functions, imaging purposes, or device-specific properties.

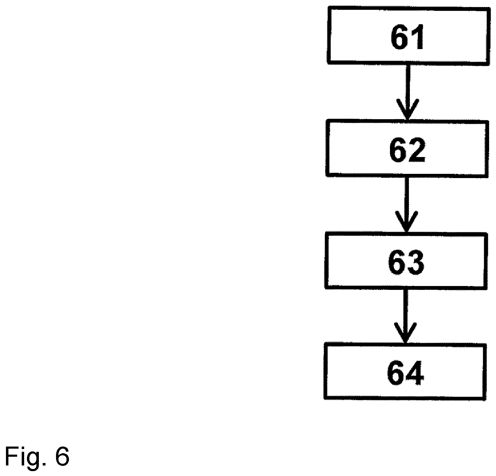

Turning to **Fig. 6****,** another embodiment of a method for operating the ultrasound system is described. The following description is based on the embodiment of the ultrasound system described above with reference to, e.g., **Fig. 4****,** but other embodiments of the ultrasound system 1 may be suited as well.

In a first step 61 of the method, a measuring mode of the ultrasound system is active. Thus, the system is configured to perform measurements using the transducer array 3.

In particular, the measurements are controlled by a system clock, which is comprised by the control unit 40. This system clock provides a frequency at which measurement steps such as generating ultrasound pulses and receiving ultrasound signals, as well as processing and analyzing of collected data are carried out.

In a step 62, the control unit 40 receives a sleep signal and subsequently activates a sleep mode of the system. According to the embodiment, the sleep signal is generated after a predetermined time duration, wherein no measurements were carried out by the system and/or wherein no data queries were received. In another embodiment, the sleep signal is received from an external unit via the interface module 11.

In response to the sleep signal, a sleep-on sequence is performed, and during this sequence, the control unit 40 shuts down a series of modules, except those that are necessary to power up the system in a later step. In particular, a wake-up controller module, an interrupt controller module, and/or a timer module are kept running during the sleep mode.

With the sleep mode activated, the system clock is configured to set a standby frequency to a value between 25 and 50 kHz, preferably to a value of 32 kHz.

In another step 63, a wake-up signal is received and the control unit 40 is configured to activate the measuring mode of the system. To this end, a sleep-off sequence is performed, wherein modules for performing measurements and providing data are subsequently started. In particular, the sleep-off sequence may be configured such that those modules, which were deactivated during the sleep-on sequence, are activated again.

According to the embodiment, the first module to be activated comprises a phase-locked loop (PLL), which is configured to provide a measurement frequency signal to the system clock. Thus, the system will advantageously be provided with a high frequency clock after performing the sleep-off sequence.

With the measuring mode activated, the system clock is configured to set a measurement frequency to a value of about 200 MHz, preferably between 150 and 250 MHz.

In a further step 64, the system 1 is configured to perform periodic measurements for monitoring the cardiac activity of a patient. These measurements are timed by the system clock, which is at a higher frequency value and allows high-frequency measurements.

In one embodiment, a common system clock is used to provide the low or high frequency in sleep and measuring mode, respectively. Thus, when the measuring mode is activated, the system clock is set to the measuring frequency, and when the sleep mode is activated, the system clock is set to the sleep frequency.

In another embodiment, a low frequency system clock and a high frequency system clock are provided. Therein, the low frequency system clock has the sleep frequency, while the high frequency system clock is set to the higher measuring frequency, once the measuring mode is activated.

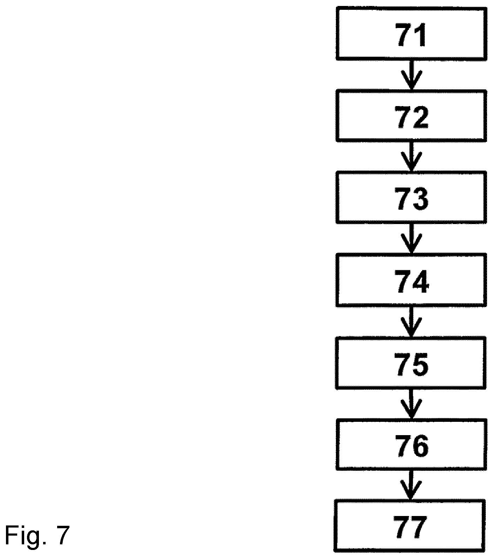

Turning to **Fig. 7**, another embodiment of a method for operating the above-described ultrasound system 1 is described. The following description is at least in principal based on the embodiment of the ultrasound system 1 described above with reference to, e.g., **Fig. 4****,** but other embodiments of the ultrasound system 1 may be suited as well.

The method comprises the steps 71-77.

In the system 1 of the embodiment, steps 72 and 73 are obtained by the reference module 45, step 74 is obtained by the artifact determination module 48, step 75 is obtained by the phase determination module 49, step 76 is obtained by the volume reconstruction module 46 and step 77 is obtained by the selected monitoring unit 47.

The respective modules/units are comprised by the control unit 40 of the ultrasound system 1.

The steps 72, 73, 74, 75 76 and/ or 77 may be performed synchronously. Further, steps 72-77 can be performed in a different order as mentioned below.

The steps 72-77 may be performed in a cyclic manner, i.e. they may be repeated for example at given intervals or triggered by predefined events and/or control signals.

In a step 71, a transducer array 3 with a plurality of transducer array elements 3a for providing ultrasound waves is placed on the body of a subject.

In a step 72, a reference ultrasound image is obtained by scanning.

In a step 73, a target organ and/or part of a target organ is detected (in this embodiment, the left ventricle).

In this embodiment, step 72 comprises two different phases of scanning which differ in terms of resolution. In particular, step 72 is characterized by starting with a faster scanning (low-resolution scanning), which is sufficient for detecting the general region of interest. A more detailed scanning of this region of interest follows, with a higher resolution, to more accurately detect e.g. features, artifacts and/or regions which need to be monitored (i.e. the region of the left ventricle). In general, there may be more than two different phases of scanning differing in terms of resolution. Also, a constant increase of resolution may be generally possible.

Based on the results of step 72, the left ventricle is detected in step 73.

In a step 74, possible artifacts such as rips and/or air bubbles between the transducer array 3 and the skin of the subject are identified. Obstructions to ultrasound signals caused by such artifacts may be avoided by reconfiguration of a sub-array 4, cf. **Fig. 4****.**

In a step 75 cardiac cycle phases are determined, in particular the end-systolic and the end-diastolic phase.

In a step 76, the volume of the left ventricle is reconstructed.

In the embodiment, step 76 comprises scanning at least partially the volume of the left ventricle without producing an image.

The scanning may be based on ultrasound based pulsed echo measurements with focused beams, wherein ultrasound beams are beam-formed by selecting a subset of transducer array elements 3a of the transducer array 3 as a sub-array 4 within the transducer array elements 3a and applying different phases to individual and/or sets of transducer array elements 3a. Data related to a single pulse-echo measurement provides the information of two boundary points of the volume of the left ventricle, wherein the volume can be reconstructed from a point cloud based on interpolation between different points of the point cloud.

Also, the volume may be reconstructed using the sparse point cloud and determining the deformation against a more detailed reference volume which would have been collected in an earlier measurement, i.e., tracking the deformation of the reference volume over time with sparse measurements. Yet another method could use the imaging data directly to track the deformation directly against a reference image.

In a step 77, the volume of the left ventricle is monitored, only at the end-systolic and the end-diastolic phase.

Note that the target organ (here the left ventricle) may in general comprise a heart or blood vessel and the part of the target organ may comprise a ventricle or atrium. In addition to targeting an organ other volumetric features inside the body may be imaged, such as e.g. the volume of a blood inside the body due to e.g. internal hemorrhaging.

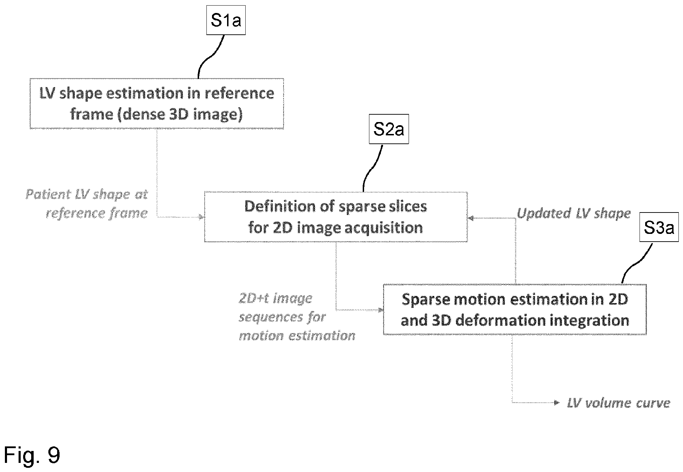

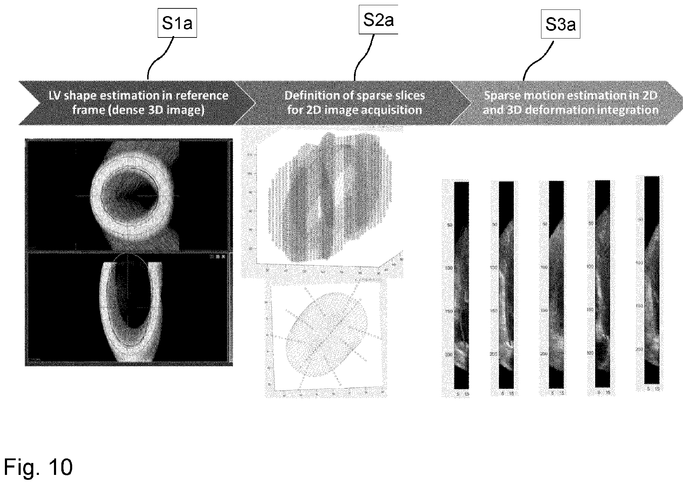

The reference module 45, volume reconstruction module 46 and monitoring unit 47 may work and interact in the following way and as shown in **Fig. 9** and **Fig. 10**:
In particular, there may be a combined sparse image acquisition and tracking for data-efficient cardiac motion estimation.

The estimation of the left ventricular (LV) stroke volume requires the identification of the LV shape and motion over the cardiac cycle.

In order to fully represent both the anatomy and function, dense characterization of the LV over the cycle is required, which typically requires the acquisition of high resolution, three-dimensional data over several time points during the cardiac cycle.

This high data acquisition throughput is usually not compatible with portable/wearable systems, which have not only limited data bandwidth but also fairly constrained power consumption requirements.

The method as shown in **Fig. 9** and in further detail in **Fig. 10** comprises three main blocks parts S1a, S2a and S3a:
The first block S1a is performing a LV shape estimation in a reference frame (dense 3D image).

It is an initial high resolution scan, which is taken for example at end-diastole, from where an existing segmentation algorithm is used to automatically delineate the LV surface (Barbosa et al, refs).

A patient LV shape at reference frame is obtained.

The second block S2a is the definition of sparse slices for 2D image acquisition.

It is an automatic selection of sub-slices for tracking based on the patient specific anatomy, where the image data over the entire cardiac cycle will be acquired, i.e. 2D image sequences over the time (2D+t images sequences) for motion estimation.

The third block S3a is a 3D deformation estimation which integrates the 2D motion estimated in each slice into a 3D affine motion model.

In order to optimize the image acquisition, the updated LV surface after its deformation has been estimated between two subsequent frame is then feedback to the sub-slice selection block, which allows to minimize the data needed to be acquire to track the LV motion.

In particular, it is advantageous to define a sparse sampling in the 3D space, through multiple 2D slices covering the regions near the left ventricular surface, where motion is estimated via an optical flow algorithm. This information is then projected to the 3D coordinate system associated with the left ventricle in order to estimate its deformation over the entire cardiac cycle. Of particular advantage is the use of a mathematical formalism that allows the efficient projection of the estimated 2D motion information onto a 3D deformation, with limited loss of performance when compared to directly estimating the 3D deformation directly from dense 3D data.

The algorithm can be described as follows: For the current estimate of the LV surface, a number of 2D slices are defined perpendicularly to the circumferential direction of the LV, where ultrasound scan lines are shot. Once data has been acquired, the 2D image gradient on each of these slices is calculated and projected to 3D by assuming image constancy along the circumferential (i.e. slice out-of-plane) dimension. The LV anatomical coordinates are then used to combine this information in a single 3D deformation matrix, via affine optical flow equations.

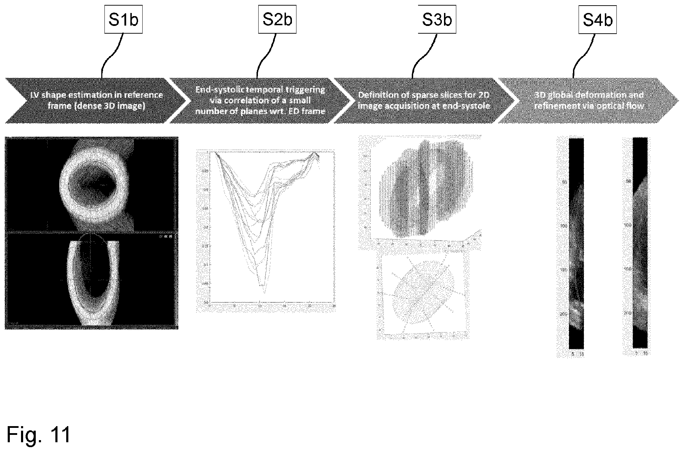

Alternatively and/or additionally, the reference module 45, volume reconstruction module 46 and monitoring unit 47 may work in the following way and as shown in **Fig. 11****:**
In particular, there may be a super sparse image acquisition and tracking for data-efficient cardiac motion estimation.

The estimation of the left ventricular (LV) stroke volume requires the identification of the LV shape and motion over the cardiac cycle. In order to fully represent both the anatomy and function, dense characterization of the LV over the cycle is required, which typically requires the acquisition of high resolution, three-dimensional data over several time points during the cardiac cycle.

This high data acquisition throughput is usually not compatible with portable/wearable systems, which have not only limited data bandwidth but also fairly constrained power consumption requirements. Considering that the estimation of multiple functional indices only require the LV shape at both end-diastole and end-systole, a potential approach to decrease the streamed data is to only scan the patient at these two time points in the cardiac cycle.

The method as shown in **Fig. 11** comprises four main blocks parts S1b, S2b, S3b and S4d:
The first block S1b is performing a LV shape estimation in a reference frame (dense 3D image).

Here, an initial high resolution scan is taken at end-diastole, from where an existing segmentation algorithm is used to automatically delineate the LV surface (Barbosa et al, refs).

The second block S2b is end-systolic temporal triggering via correlation of a small number of planes wrt. ED frame.

Here, a small number of imaging slices/planes is used to calculate the similarity of the current frame with rest to the end-diastolic one. The minimum similarity would correspond to the end-systolic frame.

The third block S3b is definition of sparse slices for 2D image acquisition at end-systole.

Here, an automatic selection of sub-slices for deformation estimation on the patient specific anatomy is performed, where the image data is acquired only at-end-systole. The fourth block S4b is 3D global deformation and refinement via optical flow.

In this block, a physiological pre-deformation is applied and then refined with the optical flow based 3D affine motion model as e.g. discussed in connection with the method of blocks S1a-S3a.

The key advantageous aspect behind the concept of the method is to combine both spatial and temporal sparsity elements to minimize data acquisition rates for the required cardiac functional indices estimation (e.g. stroke volume).

The algorithm can be described as follows:
For the current estimate of the LV surface, a small number of 2D slices (typically one or two are sufficient) are defined perpendicularly to the circumferential direction of the LV, where ultrasound scan lines are shot.

The correlation of each of these slides over time with respect to the end-diastolic frame is taken and the minimum is selected as the most likely end-systolic frame, where a larger number of 2D slices is taken.

Once data has been acquired, the 2D image gradient on each of these slices is calculated and projected to 3D by assuming image constancy along the circumferential (i.e. slice out-of-plane) dimension.

The LV anatomical coordinates are then used to combine this information in a single 3D deformation matrix, via affine optical flow equations.

In order to avoid convergence problems, a global pre-deformation of the LV is applied prior the use of the optical flow, in order to initialize the LV surface closer to the actual deformation.

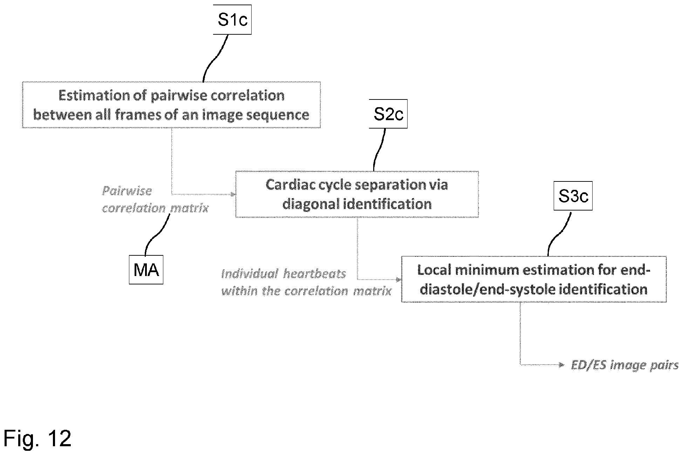

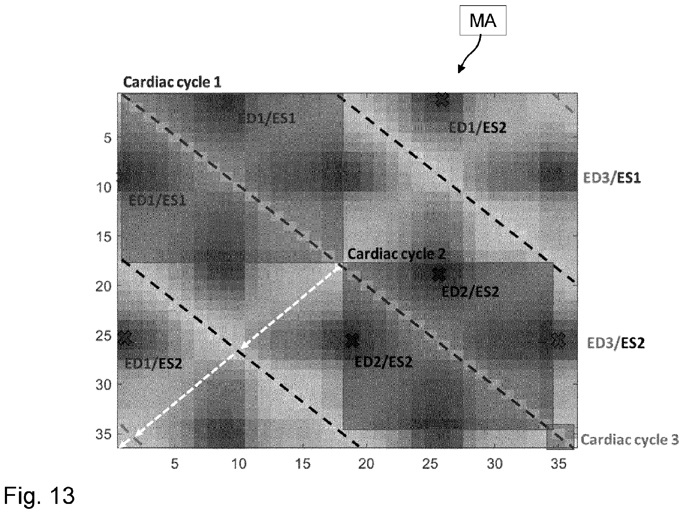

Alternatively and/or additionally, the phase determination module 49 may work in the following way and as shown in **Fig. 12** and **Fig. 13**:
In particular, there may be a reference-less automatic identification of key temporal frames in a cardiac sequence.

While conventional diagnostic ultrasound systems rely on auxiliary signals (e.g. ECG) to trigger the data acquisition over time, such signals might not be available in wearable devices whose system complexity quickly scales with the amount of different types of acquired data. Moreover, without such trigger, continuous data acquisition will stream images from multiple cardiac cycles. This implies that prior any further analysis, the sequence needs to be partitioned into individual cycles in order to estimate the relevant functional indices (e.g. stroke volume) in each heartbeat.

The method comprises three main blocks parts S1c, S2c and S3c as shown in **Fig. 12** and **Fig. 13**:
The first block S1c is estimation of pairwise correlation between all frames of an image sequence.

After a sequence of images has been acquired over a period of time, the correlation between individual frames is computed to all possible image pairs in the sequence, generating a matrix MA (cf. **Fig. 13**). In other words, the pairwise correlation matrix is provided.

The second block S2c is cardiac cycle separation via diagonal identification. The number of individual cardiac cycles is estimated via the identification of strong diagonal lines in the matrix MA.

In particular, the individual heartbeats within the correlation matrix are identified.

The third block S3c is local minimum estimation for end-diastole/end-systole (ED/ES) identification. Within each cycle, the minimum correlation is taken, corresponding to the most likely image pair representing simultaneously the end-diastolic and end-systolic frames.

End-diastole/end-systole (ED/ES) images are the result.

While inter-frame correlation has been proposed in the past for end-systolic frame identification, it always relies on the existence of a reference frame for the end-diastolic moment, which is in hand typically defined from the QRS identification on the auxiliary ECG signal in conventional ultrasound systems. Our proposed method removes the need of such reference, enabling fully automatic partitioning of image sequences into individual cardiac cycles, plus identifying the most likely frames for the end-diastolic/end-systolic image pairs.

The algorithm can be embodied as follows: Once a sequence of images has been acquired over a period of time, the correlation between individual frames is computed to all possible image pairs (i,j) in the sequence. This results in a 2D matrix M whose (i,j)th element is the correlation between the ith and the jth images in the sequence. By detecting diagonal lines, one can separate the individual heartbeats - taking advantage of the periodicity of the cardiac motion. After this, the minimum correlation is taken for each individual cardiac cycle, corresponding to the most likely image pair representing simultaneously the end-diastolic and end-systolic frames, whose mismatch is maximal. If needed, pairs between end-diastolic/end-systolic frames from different cycles can also be estimated as local minima outside each cardiac cycle (e.g. for gated image reconstruction).

### Reference numerals

- 1: Ultrasound system
- 2: Adhesive patch
- 3: transducer array; pMUT array
- 3a: transducer array element; pMUT element; transducer array unit
- 3b: sub-element; pMUT sub-element
- 3c: transducer array set
- 4: sub-array
- 5: thin-film transistor (TFT) array
- 6: TFT driver module
- 7: analog-digital converter (ADC)
- 8: pulser module
- 9: digital application-specific integrated circuit (ASIC)
- 10: Analog Front-End
- 11: interface module
- 11a: interface control module ASIC
- 12: external unit; back-end unit
- 13: memory module (RAM)
- 14: power management module
- 20: system
- 21: transducer array
- 22: analog signals
- 23: switch driver unit
- 24: receiver unit
- 25: pulser unit
- 26: signal processing; control unit
- 27: control module
- 30: micro beam-former
- 31: analog output channel
- 32: delay unit
- 33: sum unit
- 34: analog-digital converter (ADC)
- 35: digital output channel
- 40: control unit
- 41: data processing unit
- 42: complexity reduction module
- 43: deformation correction module
- 44: correction module
- 45: reference module
- 46: volume reconstruction module
- 47: selected monitoring unit
- 48: artifact determination module
- 49: phase determination module
- 51: step
- 52: step
- 61: step
- 62: step
- 63: step
- 64: step
- 71: step
- 72: step
- 73: step
- 74: step
- 75: step
- 76: step
- 77: step
- 81: step
- 82: step
- 83: step
- 84: step
- 201: system
- 202: patch case
- 203: transducer array
- 205: TFT array
- 206: beam-former module
- 209: control device
- 210: flexible PCB
- 211: USB connector
- 211a: USB transceiver
- 212: PC
- 213: RAM module
- 214: power management IC
- 301: system
- 302: flexible packaging; patch case
- 303a: sensor unit
- 306: ASIC (AFE) element
- 309: control device
- 310: flexible PCB
- 311: wireless transceiver
- 313: memory module
- 314: battery gauge
- 314a: wireless/wired charger
- 315: power IC
- 401: system
- 403: sensor
- 404: interconnects
- 406: periphery
- 406a: ICs
- 410: flexible PCB
- 414: flexible battery
- 420: acoustic matching and glue layer
- N: number of rows
- M: number of columns

- MA: matrix

- S1a: (functional) block
- S2a: (functional) block
- S3a: (functional) block
- S1b: (functional) block
- S2b: (functional) block
- S3b: (functional) block
- S4b: (functional) block
- S1c: (functional) block
- S2c: (functional) block
- S3c: (functional) block

## Claims

1. An ultrasound system (1), in particular for cardiac monitoring, comprising
- a transducer array (3) with a plurality of transducer array elements (3a) for providing ultrasound waves and detecting reflected ultrasound waves;
- a control unit (40), which is coupled to the transducer array (3), for controlling the transducer array (3),
- a data processing unit (41) for receiving data from the transducer array (3) and processing the received data,
wherein the system further comprises
- at least one reference module (45) configured for obtaining reference ultrasound data,
- at least one volume reconstruction module (46) configured for reconstructing the volume of a target organ or part of a target organ,
- at least one selected monitoring unit (47) configured for monitoring the volume of the target organ and/or part of the target organ.

2. Ultrasound system (1) according to claim 1,
**characterized in that**
the system further comprises at least one phase determination module (49) configured for determining cardiac cycle phases including at least one of start systolic phase, end systolic phase, start diastolic phase and end diastolic phase.

3. Ultrasound system (1) according claim 1 or claim 2,
**characterized in that**
the system further comprises and an artifact determination module (48) configured for identification of artifacts and avoiding obstructions to ultrasound signals caused by artifacts.

4. A method for ultrasound imaging of a body part of a subject comprising at least the following steps:
- Placing a transducer array (3) with a plurality of transducer array elements (3a) for providing ultrasound waves for transmission into the body of the subject;
- Obtaining reference ultrasound data by scanning, wherein the reference ultrasound data are based on reception of the ultrasound waves transmitted into the body of the subject;
- Detecting a target tissue or anatomical structure and/or part of a target tissue or anatomical structure;
- Reconstructing the volume of the target tissue or anatomical structure and/or part of the target organ;
- Monitoring the volume of the target tissue or anatomical structure and/or part of the target tissue or anatomical structure.

5. The method according to claim 4,
**characterized in that**
the step of obtaining reference ultrasound data comprises at least two different phases of scanning, wherein the at least two different phases of scanning differ in terms of resolution.

6. The method according to claim 4 or claim 5,
**characterized in that**
the method further comprises the step of identifying artifacts and avoiding obstructions to ultrasound imaging caused by artifacts.

7. The method according to one of claims 4 to 6,
**characterized in that**
reconstructing the volume of the target tissue or anatomical structure and/or part of the target tissue or anatomical structure comprises scanning at least partially the volume of the target tissue or anatomical structure and/or blood vessel without producing an image.

8. The method according to one of the claims 4 to 7,
**characterized in that**
the method further comprises the step of determining cardiac cycle phases including at least one of start systolic phase, end systolic phase, start diastolic phase and end diastolic phase, wherein the volume monitoring is performed only at one or more cardiac cycle phases.

9. The method according to claim 8,
**characterized in that**
the cardiac cycle phases are determined by sending at least one ultrasound beam through (a) defined position(s) of the target organ and/or part of the target organ at least two times through at least one cardiac cycle.

10. The method according to any of the claims 4 to 9,
**characterized in that**
the method comprises combined sparse image acquisition and tracking for data-efficient cardiac motion estimation.

11. The method according to claim 10,
**characterized in that**
the method comprises at least the following steps:
- Performing a shape estimation on a reference frame (dense 3D image);
- Defining of sparse sampling locations, such as single beams and/or slices, for 2D image acquisition; and
- Estimating a 3D deformation which integrates the 2D motion estimated in each slice into a 3D affine motion model.

12. The method according to claim 11,
**characterized in that**
the method comprises at least the following steps:
- Performing a shape estimation on a reference frame (dense 3D image);
- End-systolic temporal triggering via correlation of a small number of planes;
- Defining of sparse sampling locations, such as single beam and/or slices, for 2D image acquisition at end-systole; and
- Performing a 3D global deformation and refinement via optical flow.

13. The method according to any of the claims 4 to 12,
**characterized in that**
the method comprises reference-less automatic identification of key temporal frames in a cardiac sequence.

14. The method according to claim 13,
**characterized in that**
the method comprises at least the following steps:
- Estimating of pairwise correlation between all frames of an image sequence;
- Performing a cardiac cycle separation via diagonal identification; and
- Performing a local minimum estimation for end-diastole/end-systole (ED/ES) identification.

15. The method according to claim 11 or claim 12,
**characterized in that**
the reference frame is measured according to a two-step procedure, wherein a first step is collecting a more coarse image of the region of interest and this coarse image is used to detect the volume of interest, and wherein in a second step a detailed scanning is performed to focus on the detected volume of interest.
